(19) Europäisches Patentamt
European Patent Office
Office européen des brevets

(11) EP 1 938 841 A2

(12) EUROPEAN PATENT APPLICATION

(43) Date of publication:
**02.07.2008 Bulletin 2008/27**

(21) Application number: **07024839.8**

(22) Date of filing: **20.12.2007**

(51) Int Cl.:
***A61K 45/06*** *(2006.01)* ***A61P 33/06*** *(2006.01)*
***A61P 31/04*** *(2006.01)*

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **20.12.2006 IN LL27262006**

(71) Applicants:
- **National Institute of Immunology**
  **New Delhi 110067 (IN)**
- **INDIAN INSTITUTE OF SCIENCE**
  **A Registered Trust, in India,**
  **Under Charitable Endowments Act**
  **Bangalore 560 012 (IN)**

(72) Inventors:
- **Surolia, Avadhesha**
  **New Delhi, 110 067 (IN)**
- **Surolia , Namita**
  **Karnataka, 560 064 (IN)**
- **Sharma, Shailendra, K.**
  **Karnataka, 560012 (IN)**
- **Parasuraman, Prasanna**
  **Karnataka, 560012 (IN)**
- **Kumar, Gyanendra**
  **Karnataka, 560012 (IN)**

(74) Representative: **Ebner von Eschenbach, Jennifer et al**
**LADAS & PARRY LLP**
**Dachauerstrasse 37**
**80335 München (DE)**

# (54) Synergistic composition for modulating activity of substrate analogs for NAD+, NADP+, NADH+ or NADPH dependent enzymes and process thereof

(57) The present disclosure provides a composition for enhancing effect of an inhibitor in inhibiting $NAD^+$/ $NADP^+$ or NADH/NADPH dependent enzymes. The inhibition of the $NAD^+$/$NADP^+$ or NADH/NADPH dependent enzymes such as Enoyl-ACP reductase (ENR) by the composition of the present disclosure serves as a target for treating malaria and other infectious diseases. The present disclosure provides composition comprising inhibitor and polyphenol, wherein the polyphenol was found to enhance the inhibitory activity of the inhibitor. The present disclosure provides method for treating an infectious disease comprising administering an effective amount of the composition of the present disclosure to patients in need thereof. The present disclosure further provides a method for identifying a compound that enhances the effect of the inhibitor, a method of determining the antimalarial activity of a compound and use of polyphenol as a bioenhancer that enhances effect of an inhibitor for inhibiting $NAD^+$/$NADP^+$ or NADH/NADPH dependent enzymes. The present disclosure provides a composition comprising inhibitor and a polyphenol wherein the polyphenol enhances effect of the inhibitor for inhibiting aldose reductase for treating complications of diabetes that include diabetic retinopathy, cataract neuropathy and neural complication.

EP 1 938 841 A2

## Description

## TECHNICAL FIELD

The present disclosure relates to a composition useful for enhancing the effect of an inhibitor for inhibiting $NAD^+$, $NADP^+$, NADH or NADPH dependent enzymes.

## BACKGROUND AND PRIOR ART

Malaria is one of the most devastating diseases of tropical countries. *Plasmodium falciparum* causes the fatal kind of malaria, viz. cerebral malaria. Drug resistance in the malarial parasite to antimalarials has further worsened the current situation. Hence, there is an urgent need to explore novel pathways exclusive to the parasite for developing antimalarial agents. In this context, the discovery of a bacterial type of fatty acid synthesis pathway in *Plasmodium falciparum* has made it possible to explore new drug targets for the purpose of developing antimalarials.

Fatty acids are synthesized by either type I or type II fatty acid synthase (FAS)1 Type I or the associative type, in which a single large multifunctional protein completes the fatty acid biosynthesis is present in eukaryotes and certain mycobacteria [1]. Type II FAS (dissociative type of FAS) found in most prokaryotes, plants and protozoans including the malarial parasite is carried out by several discrete enzymes having specific reactions to accomplish fatty acid synthesis. The differences in the organization of the FAS systems of the host and parasite can therefore be utilized for the development of antimalarials.

In *Plasmodium falciparum,* fatty acid biosynthesis takes place in a relict plastid, called the apicoplast [2]. All the enzymes of FAS are encoded by nuclear genes and are transported to the apicoplast by a bipartite signal sequence. The fatty acid elongation cycle contains four iterative steps, decarboxylative condensation, NADPH dependent reduction, dehydration and NADH dependent reduction [1]. The fourth step is carried out by enoyl-acyl carrier protein (ACP) reductase (ENR), which reduces the trans-2 enoyl bond of enoyl-ACP substrates to saturated acyl- ACP.

The substrate binding loop region in an enzyme of malarial parasite *Plasmodium falciparum* enoyl-Acyl carrier protein (ACP) reductase (PfENR) is ordered both for the binary (PfENR-NADH) as well as the ternary complex (PfENR-NAD+-Triclosan) but in its *E. coli* counterpart viz EcENR the loop becomes ordered or stabilized only after the formation of ternary complex [6].

Flavonoids like Green tea extracts (GTE) are useful for treating many diseases. GTE contain a variety of secondary metabolites, mainly flavonoids called catechins, which include (-) epigallocatechin gallate (EGCG), (-) epicatechin gallate (ECG), (-) epigallocatechin (EGC) and (-) epicatechin (EC). EGCG is an effective flavonoid for treating various types of cancers and is a powerful antioxidant. Tea catechins also inhibit type II fatty acid biosynthesis in *E. coli* by inhibiting β-ketoacyl-ACP reductase and ENR with IC50 in the 5-15 μM range [7]. However, none of the prior art references suggest use of polyphenols, for example, that include flavonoids, as a bioenhancer for enhancing the effect of an inhibitor in inhibiting NAD+/NADP+ or NADH/NADPH dependent enzymes.

## SUMMARY

The present disclosure describes compositions that are useful for enhancing the effect of an inhibitor in inhibiting NAD+/NADP+ or NADH/NADPH dependent enzymes, said compositions comprise an effective amount of an inhibitor and a polyphenol. The inhibitor encompasses 2'-hydroxydiaryl ether or derivatives thereof which include 2'-hydroxydiphenyl ether which in turn encompasses compounds that include triclosan. The polyphenols potentiating the inhibitory activity of the inhibitors include flavonoids selected from a group consisting of flavonol, isoflavonol, flavanones, flavan-3-ol and derivatives thereof. Compositions of this invention can be used to treat or prophylactically treat an infectious disease such as malaria or a bacterial infection.

The present disclosure also provides a method for identifying a compound that enhances the effect of an inhibitor for inhibiting NAD+/NADP+ or NADH/NADPH dependent enzymes and can be used to treat or prophylatically treat an infectious disease such as malaria or a bacterial infection. The infectious disease may be caused by infectious agents such as bacteria and malarial parasites.

## BRIEF DESCRIPTION OF THE ACCOMPANYING DRAWING

Figure 1A depicts the inhibition of PfENR by EGCG.

Figure1B depicts the inhibition kinetics of EGCG with respect to NADH.

Figure 1C depicts the inhibition kinetics of EGCG with respect to crotonoyl CoA.

Figure 2 depicts the slow onset of inhibition by triclosan in presence of EGCG.

Figure 3A depicts the effect of EGCG on triclosan inhibition of PfENR.

Figure 3B depicts the effect of triclosan on EGCG inhibition.

Figure 4A depicts the binding of [$^3$H] EGCG to PfENR in the absence of triclosan.

Figure 4B depicts the gel filtration profile of [$^3$H] EGCG binding to PfENR.

Figure 4C depicts the binding of [$^3$H]EGCG in the presence of Triclosan. Binding was assessed using PVDF membranes.

Figure 4D depicts the influence of Triclosan on [$^3$H] EGCG binding.

Figure 5 depicts the triclosan progress curves.

Figure 6 depicts PfENR inhibition on triclosan concentration in presence of EGCG.

Figure 7 depicts the dissociation rate constant ($k_6$) of PfENR-Triclosan-EGCG complex.

Figure 8 depicts the effect of triclosan concentration on tryptophan fluorescence.

Figure 9 depicts the time-dependent fluorescence quenching of PfENR by triclosan in the presence of EGCG.

Figure 10A depicts the EGCG-PfENR and EGCG-EcENR docked complexes superimposed on each other.

Figure 10B depicts the EGCG and triclosan bound with PfENR in a modeled ternary complex.

**DETAILED DESCRIPTION**

The present disclosure provides compositions for enhancing the effect of an inhibitor in inhibiting $NAD^+/NADP^+$ or NADH/NADPH dependent enzymes that participate in type II fatty acid synthesis. The inhibition of the $NAD^+/NADP^+$ or NADH/NADPH dependent enzymes that include Enoyl-ACP reductase (ENR) serve as a target. Since all bacteria and many apicomplexan parasites such as the malarial parasite are dependent on type II fatty acid synthesis pathway of which ENR is a key enzyme, it is a validated target for the development of anti-bacterial and anti-malarial agents[1,2]. These agents are used for treating malaria and other infectious diseases. The present disclosure provides a method for identifying a compound that enhances the effect of an inhibitor for inhibiting NAD+/NADP+ or NADH/NADPH dependent enzymes, said method comprising incubating the NAD+/NADP+ or NADH/NADPH dependent enzyme with an inhibitor, a compound and enzyme substrate to form a ternary complex; and determining the inhibition of the NAD+/NADP+ or NADH/NADPH dependent enzyme.

A composition according to the invention comprises an inhibitor and a polyphenol wherein polyphenol potentiates the inhibitory activity of the inhibitor. The composition comprises amounts of an inhibitor and polyphenol that are effective to treat malaria or other infectious diseases.

Inhibitors that can be used include 2'-hydroxydiaryl ether or derivatives thereof such as 2'-hydroxydiphenyl ether or derivatives thereof. An example of an inhibitor is triclosan.

Polyphenols include flavonoids such as quercetin; flavonol; isoflavonol; flavanones; flavan-3-ol such as catechin; buteine; and derivatives thereof.

The compositions may include a catechin selected from epigallocatechin gallete (EGCG), epigallocatechin (EGC) and epicatechin gallete (ECG).

Polyphenols include catechins, quercitin and butein that enhance the inhibitory activity of triclosan against *Plasmodium falciparum* enoyl-Acyl carrier protein (ACP) reductase (PfENR), *in vitro P. falciparum* culture and *in vivo P. berghei* culture in mouse malarial model.

Tea catechin enhances the inhibitory activity of triclosan against *Plasmodium falciparum* enoyl-Acyl carrier protein (ACP) reductase (PfENR), in vitro *P. falciparum* culture and in vivo *P. berghei* culture in mouse malarial model.

In addition to the inhibitor and polyphenol, the compositions may also include an antimicrobial compound that

acts by forming a ternary complex with $NAD^+$, $NADP^+$ NADH or NADPH

Non-limiting examples of antimicrobial compounds are antibacterial compounds, antimalarial compounds, antiprotozoal compounds, antifungal compounds, antiproliferative compounds and analgesics.

The present disclosure also provides use of polyphenol as a bioenhancer where the polyphenol enhances the effect of an inhibitor for inhibiting $NAD^+$/$NADP^+$ or NADH/NADPH dependent enzyme of Apicomplexa species, *Plasmodium* species or bacterial species. The NAD+/NADP+ or NADH/NADPH dependent enzymes are those of an Non-limiting examples of the *Plasmodium* species are t hose of *Plasmodium falciparum, Plasmodium vivax, Plasmodium ovale* and *Plasmodium malaria.*

Non-limiting examples of bacterial species are those of *Escherichia coli* and *Mycobacterium tuberculosis.*

The present disclosure also describes the use of polyphenol as an bioenhancer, wherein the polyphenol enhances the inhibitory activity of triclosan, its derivatives, analogues or pharmaceutically acceptable salt against *Plasmodium falciparum* enoyl-Acyl carrier protein (ACP) reductase (PfENR). This is described in relation to *in vitro P. falciparum* culture and *in vivo P. berghei* culture in a mouse malarial model.

Another embodiment of the present disclosure provides a method for identifying a compound that enhances the effect of an inhibitor for inhibiting $NAD^+$/$NADP^+$ or NADH/NADPH dependent enzymes said method comprising incubating the $NAD^+$/$NADP^+$ or NADH/NADPH dependent enzyme with an inhibitor, a compound and enzyme substrate to form a ternary complex; and determining the inhibition of the $NAD^+$/$NADP^+$ or NADH/NADPH dependent enzyme. Examples of such enzymes are *Plasmodium falciparum* enoyl-Acyl carrier protein (ACP) reductase (PfENR), *E. coli* enoyl-Acyl carrier protein (ACP) reductase (EcENR), aldolase reductase, steroid 5$\alpha$-reductase, bacterial FabG, dihydrofolate reductase and squalene epoxidase.

Another embodiment of the present disclosure provides a composition useful for enhancing effect of an inhibitor in inhibiting $NAD^+$/$NADP^+$ or NADH/NADPH dependent enzymes where the $NAD^+$/$NADP^+$ or NADH/NADPH dependent enzymes. Non-limiting examples of such enzymes are selected from *Plasmodium falciparum* enoyl-Acyl carrier protein (ACP) reductase (PfENR), *E. coli* enoyl-Acyl carrier protein (ACP) reductase (EcENR), aldolase reductase, steroid 5$\alpha$-reductase, *P. falciparum* FabG , bacterial FabG, dihydrofolate reductase and squalene epoxidase.

In embodiment of the invention, the enzyme is PfENR or a bacterial ENR EcENR.

Another embodiment of the present invention relates to a composition for enhancing the inhibitory activity of $NAD^+$, $NADP^+$, NADH or NADPH dependent enzymes by combining the use of polyphenols capable of binding to the enzyme close to the active site thereby forming a ternary complex. The inhibitors include 2'-hydroxydiaryl ether or derivatives thereof, preferably 2'-hydroxydiphenyl ether or derivatives thereof, more preferably triclosan.

Examples of ternary complexes include a complex formed of *Plasmodium falciparum* enoyl-Acyl carrier protein (ACP) reductase (PfENR), triclosan or its derivatives or analogues, or hydroxydiphenyl ether and polyphenol exemplified by quercetin wherein the overall inhibition constant is $280.6 \pm 11.78$pM.

The inhibitor inhibits enzymes that use $NAD^+$ or $NADP^+$ or NADH or NADPH as a cofactor by promoting a ternary complex formation between the inhibitor which is an analog of the cofactor (I), their substrate(s) or an analog of the substrate (A) or the substrate (s) itself and such enzymes (E). Essentially in this complex both the inhibitor or the substrate analog in its ternary complex (I-A-E or S-A-E complex) are bound so tightly because of which inhibitor (I) or the substrate analog (A) barely dissociates from the ternary complexes such as E-A-I or E-A-S. The formation of such a ternary complex thereby completely inhibits the target enzyme.

Substrate analogs are molecules that can be recognized and bound by an enzyme because they sufficiently resemble that enzyme's substrate. However, the enzyme is unable to affect the analog in the same manner it can affect its natural substrate. Thus substrate analogs can be efficient inhibitors of a given catalytic process and can provide unique opportunities for the study of biologically active molecules.

A composition comprising an inhibitor and polyphenol leads to ternary complex formation with polyphenols, wherein the inhibitor or substrate analog (A) for inhibiting the enzymes involved in fatty acid synthesis of malarial parasite and bacteria and thereby inhibiting their growth is a 2'-hydroxydiphenyl ether, 2'-hydroxydiaryl ether or a derivative thereof as represented by Formula 1,

Formula 1

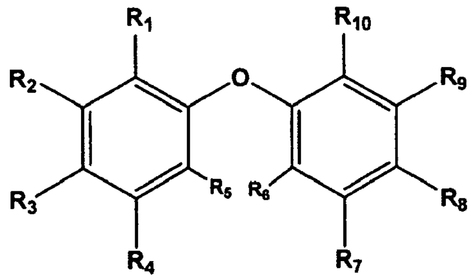

In Formula 1, $R_1$, $R_2$, $R_3$, $R_4$, $R_5$, $R_6$, $R_7$, $R_8$, $R_9$, and $R_{10}$ are independently selected from H, OH, CHO, $CH_3$, $OCH_3$, Cl, $NO_2$, $NH_2$, $NHCOCH_3$, COOH, $COOCH_3$, $CH_2OH$, $CH_2Cl$, CN, $OCH_2CH_3$, $OCOCH_3$ and $CH_2NHCH_2CH_3$ and either $R_1$ or $R_{10}$ has to have a hydroxyl (OH) group.

Surprisingly, a compound similar to that of Formula 1 that has a -S or -$CH_2$ moiety in place of the central -0 group does not have an inhibitory effect on the NAD, NADP, NADH or NADPH dependent enzymes. Therefore, such compounds are not encompassed by the term "inhibitor" of the present disclosure.

The inhibitor or substrate analog (A) could also be a 2'-hydroxydiphenyl ether, more specifically triclosan (2', 4,4'-trichloro-2-hydroxydiphenyl ether or 2,4,4"-trichloro-2'-hydroxydiphenyl ether or 5-chloro-2-(2, 4-dichlorophenoxy) phenol). The structure of triclosan (TCL) is provided below as Formula 2.

Formula 2

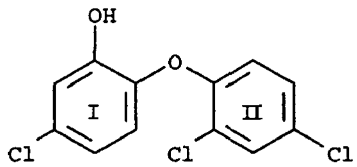

The polyphenols include flavonols or 3-hydroxy-2-phenyl-4H-chromen-4-one as given by Formula 3 as bioenhancers which are also the inhibitors (viz. I) for increasing the inhibitory potency of $NAD^+$/$NADP^+$/NADH/NADPH dependent enzymes exemplified by PfENR, EcENR by substrate analogs (A) consisting of hydroxydiphenyl ethers (compounds with Formulas 1 and 2) or glyceraldehydes-a substrate analog for aldose reductase enzyme; *Plasmodium falciparum* enoyl-Acyl carrier protein (ACP) reductase (PfENR) and E. coli enoyl ACP-reductase (EcENR). Likewise for, aldose reductase (where S= glyceraldehydes or another aldose), steroid 5α-reductase, *E. coli* or P. *falciparum* FabG [EcFabG/PfFabG] (where S= acetoacetyl-CoA or acetoacetyl-Acyl Carrier Protein), dihydrofolate reductase (where S= dihydrofolate), squalene epoxidase (where S= squalene) etc.

Formula 3

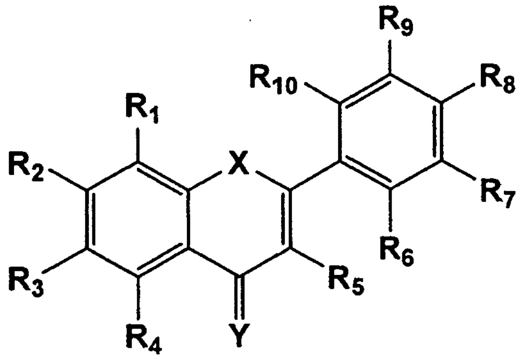

In Formula 3, X and Y are independently selected from O, NH, S and $CH_2$; and $R_1$, $R_2$, $R_3$, $R_4$, $R_5$, $R_6$, $R_7$, $R_8$, $R_9$, and $R_{10}$ are independently selected from H, $CH_3$, OH, $OCH_3$, $OCH_2CH_3OCOCH_3$. $NH_2$, CHO, Cl, $CH_2OH$, $CH_2Cl$, CN, COOH and $COOCH_3$.

The polyphenols also include isofavonols, 3- 4H-chromen-4-one or a derivative represented as Formula 4 as bioenhancers which are also described here as inhibitors (I) for increasing the inhibitory potency of $NAD^+$/$NADP^+$/NADH/NADPH dependent enzymes for compounds with Formulas 1 and 2; *Plasmodium falciparum* enoyl-Acyl carrier protein (ACP) reductase (PfENR), E. coli enoyl ACP-reductase (EcENR), aldolase reductase, aldose reductase (where A= glyceraldehydes or an aldose), steroid 5α-reductase, bacterial FabG (where A= acetoacetyl-coenzymeA / acetoacetyl-ACP) and Fabl reductases, dihydrofolate reductase (where A= dihydrofolate), squalene epoxidase (where A= squalene) etc.

Formula 4

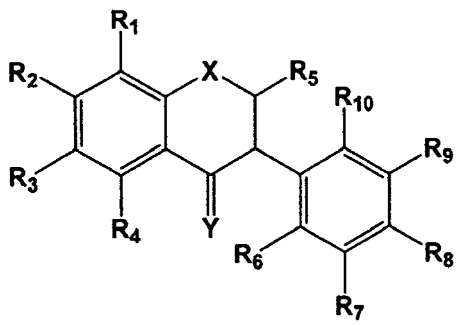

In Formula 4, X and Y are independently selected from O, NH, S or $CH_2$ and $R_1$, $R_2$, $R_3$, $R_4$, $R_5$, $R_6$, $R_7$, $R_8$, $R_9$, and $R_{10}$ are independently selected from H, $CH_3$, OH, $OCH_3$, $OCH_2CH_3$, $OCOCH_3$, $NH_2$, CHO, Cl, $CH_2OH$, $CH_2Cl$, CN, COOH and $COOCH_3$.

The polyphenols also include flavanones or 2,3-Dihydro-2-phenylchromen-4-one represented below as Formula 5 as bioenhancers which are also described here as inhibitors (I) for increasing the inhibitory potency of $NAD^+$/$NADP^+$/NADH/NADPH dependent enzymes; *Plasmodium falciparum* enoyl-Acyl carrier protein (ACP) reductase (PfENR), E. coli enoyl ACP-reductase (EcENR), aldolase reductase, aldose reductase (where A= glyceraldehyde), steroid 5α-reductase, EcFabG and PfFabG and Fabl reductases, dihydrofolate reductase, squalene epoxidase etc.

Formula 5

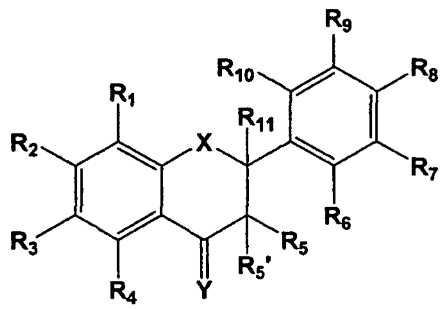

In Formula 5, X and Y are independently selected from O, NH, S and $CH_2$ and $R_1$, $R_2$, $R_3$, $R_4$, $R_5$, $R_5$, $R_6$, $R_7$, $R_8$, $R_9$. $R_{10}$ and $R_{11}$ are independently selected from H, $CH_3$, OH, $OCH_3$, $OCH_2CH_3$, $OCOCH_3$, $NH_2$, CHO, Cl, $CH_2OH$, $CH_2Cl$, CN, COOH and $COOCH_3$.

Another embodiment of the present disclosure provides a composition for enhancing the inhibitory activity of $NAD^+$, $NADP^+$, NADH or NADPH dependent enzymes comprising inhibitor and polyphenol. The polyphenol includes Flavan-3-ols or 3,4-dihydro-2-phenyl-2H-chromen-3-ol represented below as Formula 6 as bioenhancers which are also described here as inhibitors, I, for increasing the inhibitory potency of $NAD^+$/$NADP^+$/NADH/NADPH dependent enzymes for example, *Plasmodium falciparum* enoyl-Acyl carrier protein (ACP) reductase (PfENR), E. coli enoyl ACP-reductase (EcENR), aldolase reductase, human steroid 5α-reductase, bacterial FabG and FabI reductases, dihydrofolate reductase, squalene epoxidase etc.

Formula 6

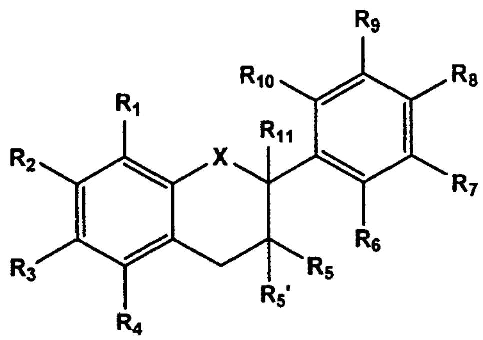

In Formula 6, X is selected from O, NH, S or $CH_2$. $R_1$, $R_2$, $R_3$, $R_4$, $R_6$, $R_7$, $R_8$, $R_9$, $R_{10}$ and $R_{11}$ are independently selected from H, $CH_3$, OH, $OCH_3$, $OCH_2CH_3$, $OCOCH_3$. $NH_2$, CHO, Cl, $CH_2OH$, $CH_2Cl$, CN, COOH and $COOCH_3$, or

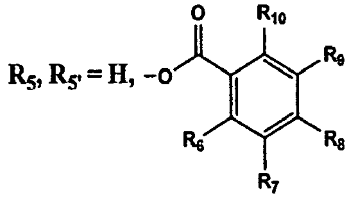

In another embodiment the inhibitory activity of the inhibitor or the substrate analogs (A) represented by Formulas 1 and 2, their derivatives, analogues or pharmaceutically acceptable salt thereof is enhanced by mimicking the triclosan-PfENR-NAD complex in the presence of triclosan-PfENR-catechin complex or triclosan-PfENR-polyphenol complex.

In another embodiment the polyphenol is a tea catechin that acts as a bioenhancer that enhances the inhibitory activity of compounds with Formulas 1 and 2 against the enzyme, *Plasmodium falciparum* enoyl-acyl carrier protein (ACP) reductase (PfENR). The inhibition of the enzyme PfENR inbits the growth of malaria parasite *in vitro* cultures also leading to the treatment of malaria based on *in vitro* or *in vivo* mouse model of the disease.

The present disclosure also describes compositions that are useful for enhancing the effect of an inhibitor in inhibiting $NAD^+/NADP^+$ or NADH/NADPH dependent enzymes which participate in the fatty acid synthesis of infectious agents.

The inhibition of the growth of the malarial parasite following administration of the composition of the present disclosure can be monitored by monitoring the incorporation of [$^3$H] hypoxanthine in nucleic acid as a quantitative indicator of the inhibition of the parasite growth; and examining smears of treated cultures for morphological features of the parasite as an indicator of growth.-Example 2 is an example of this method.

The effect of a compound to inhibit the elongation of fatty acid synthesis in a malarial parasite, specifically the enoyl ACP reductase enzyme and thus determine its antimalarial activity can be measured. The method comprises the spectrophotometric measurement of its activity using enoyl-CoA, enoyl-ACP, enoyl ACP reductase or other intermediates of fatty acid synthesis as substrates.

The antimalarial activity of a compound can also be measured by detecting the product of the enzymatic reaction following the separation of the substrate and product by reverse phase-HPLC.

The infectious disease may be caused by infectious agents such as bacteria and malarial parasites.

The present disclosure also describes a composition useful for enhancing effect of an inhibitor in inhibiting aldose reductase, said composition comprising an effective amount of inhibitor and a polyphenol.

In still another embodiment, the present disclosure provides a composition for enhancing the inhibitory activity of NAD, NADP, NADH or NADPH dependent enzymes comprising inhibitor and polyphenol such as aldose reductase by ternary complex formation between aldose reductase-flavonoids-glyceraldehyde where glyceraldehyde is a substrate analog. Kd of glyceraldehyde decreased from 510 μM to 40 μM in the presence of 2 μM of the polyphenol quercetin by more than 10 fold. The dissociation constant of quercetin also decreased significantly from 270 nM to 30 nM in the presence of 75 μM of glyceraldehyde. The formation of such a complex therefore can be successfully utilized to inhibit aldose reductase and thus to combat chronic complications of diabetes that includes cataract, retinopathy, neuropathy and nephropathy.

The term dissociation constant is a specific type of equilibrium constant that measures the propensity of a larger object to separate (dissociate) reversibly into smaller components, as when a complex falls apart into its component molecules, or when a salt splits up into its component ions. The dissociation constant is usually denoted $K_d$ and is the inverse of the affinity constant. In the special case of salts, the dissociation constant can also be called ionization constant.

The present disclosure also provides use of polyphenol as a bioenhancer where the polyphenol enhances effect of an inhibitor for inhibiting $NAD^+/NADP^+$ or NADH/NADPH dependent enzymes of bacterial species such as *Escherichia coli.*

The triclosan-PfENR-catechin ternary complex or triclosan-PfENR-polyphenol ternary complex enhances the inhibitory activity of triclosan or its derivatives or analogues or pharmaceutically acceptable salt thereof against *Plasmodium falciparum* enoyl-Acyl carrier protein (ACP) reductase (PfENR).

The present disclosure provides the overall inhibition constant for triclosan (a representative of substrate analogs (A) with formula 1 or 2 against PfENR with buteine is 84.0 pM.

In another embodiment, the present disclosure provides the surprising result of IC50 for the growth of malaria culture in red blood cells for EGCG is 191.μM, ECG is 4.7 μM, EGC is 210.9 μM, Quercetin is 20.7 μM and Butein is 49.7 μM.

In another embodiment the present disclosure provides that administration of a flavonoid increases the survival time of animals suffering with systematic bacterial infections.

The present disclosure provides a composition comprising inhibitor and polyphenol wherein the polyphenol is medicinally important secondary metabolites found in the most commonly consumed beverage like tea that inhibit enoyl-ACP reductase (PfENR) of *Plasmodium falciparum* at nanomolar concentrations. They also inhibit P. *falciparum* growth in culture with $IC_{50}$ in low micromolar (4.7 μM for ECG, 20.7 μM for quercetin) range. Moreover, mice treated with 30-40mg/kg of tea catechins survived for prolonged periods of time (10-12 days) as compared to their untreated counterparts which died within 6 days of infection. These tea compounds also inhibit the mammalian FAS but with Ki in micromolar range, which is far greater than the *Plasmodium* counterpart. Hence, the mammalian FAS is 10,000 times poorly inhibited as compared to the *Plasmodium falciparum* FAS. The huge difference in Ki of EGCG between the host and the parasite ENR underscores the significance of present disclosure.

The binding of triclosan is potentiated by flavonoids and vice-versa by the formation of E-I-A ternary complex by steady-state kinetics and also by [$^3$H] EGCG binding experiments. It was surprisingly found that of overall inhibition constant of triclosan in presence of EGCG turns out to be in the low picomolar range (2 pM to 280pM depending on the method of analysis) and this process followed a biphasic mode with slow tight binding mechanism. Fluorescence titration

and time course experiments for the binding of triclosan, a representative of a series of compounds were in agreement with the kinetic data. The biochemical data are supported by docking studies where it is confirmed that these tea catechins occupy the cofactor binding site of PfENR and thus verify the formation of the stable ternary complex. As a result flavonoids/tea catechins, especially EGCG can be used for the formulation of antimalarial therapy in conjugation with triclosan or another 2'-hydroxydiphenyl ether or 2'-hydroxydiaryl ether as a combination drug.

In an embodiment, the present disclosure provides method to make a flavonoid specific to PfENR to chemically manipulate the essential galloyl scaffold of these catechins by rational drug design using PfENR structure. This method can be used to fight against malaria, bacterial infection, complications of diabetes etc. as the tea catechins are non-toxic, inexpensive and abundant in nature. This method can be extended to a number of $NAD^+$/$NADP^+$ dependent enzymes like aldose reductase to fight cataract formation as a complication of diabetes, steroid 5$\alpha$-reductase, bacterial FabG and Fabl reductases, dihydrofolate reductase for combating both infectious diseases and cancer.

The use of flavonoids (I) leads to the enhancement in the inhibition of *Plasmodium falciparum* enoyl-Acyl carrier protein (ACP) reductase (PfENR) as well as against *E. coli* enoyl ACP-reductase (EcENR) by triclosan an analog of the substrate (A) for this enzyme because of the ternary complex formation between PfENR or EcENR, I and A or S was demonstrated. The applicability of the method of potentiating inhibition of aldose reductase (aR) by ternary complex formation (aR-I-A) between aR, I (a flavanoid) and A (glyceraldehyde) is demonstrated.

Catechins potentiating triclosan by binding to PfENR at levels not observed with $NAD^+$ [14], bringing the overall inhibition constant of triclosan and other 2'-hydroxydiphenyl ethers in the picomolar range. The overall inhibition constant Ki* varied in the range of 1.9 $\pm$ 0.46 to 280 pM for PfENR with the different polyphenol used. There was a higher inhibitory activity of the tea catechins towards PfENR observed. EGCG in conjugation or in combination with triclosan or its analogs can be used for developing more effective antimalarials. It was also observed that EGCG potentiates the inhibition of E. coil enoyl ACP-reductase by triclosan (A) and other 2'-hydroxydiaryl ethers that includes 2'-hydroxydiphenyl ether. This serves as a mode for treating broad spectrum of bacterial infections or wherever a ternary complex formation between flavonoids or a tea catechins defined here as I and an enzyme substrate or its analogs (A) with the enzyme can be formed. This method can be extended to several other $NAD^+$/$NADP^+$ dependent enzymes like aldolase reductase, steroid 5$\alpha$-reductase, bacterial FabG and Fabl reductases, dihydrofolate reductase, squalene epoxidase and glutamate dehydrogenase.

In some of the *in vitro* experiments of the invention, the inhibitor has a dosage ranging from 1.0 pM to 100 $\mu$M and the polyphenol has a dosage ranging from 100 pM to 100 $\mu$M.

The composition of the present disclosure may be administered in a therapeutically effective amount to a subject such as a mammal such as a human. Therapeutically effective amounts of the composition of the present disclosure may be used to treat, modulate, attenuate, reverse, or affect malaria in a mammal.

As used herein, the term "treating" refers to reversing, alleviating or inhibiting the progress of a disease, disorder or condition, or one or more symptoms of such disease, disorder or condition, to which such term applies. As used herein, "treating" may also refer to decreasing the probability or incidence of the occurrence of a disease, disorder or condition in a mammal as compared to an untreated control population, or as compared to the same mammal prior to treatment. For example, as used herein, "treating" may refer to preventing a disease, disorder or condition, and may include delaying or preventing the onset of a disease, disorder or condition, or delaying or preventing the symptoms associated with a disease, disorder or condition. As used herein, "treating" may also refer to reducing the severity of a disease, disorder or condition or symptoms associated with such disease, disorder or condition prior to a mammal's affliction with the disease, disorder or condition. Such prevention or reduction of the severity of a disease, disorder or condition prior to affliction relates to the administration of the composition of the present invention, as described herein, to a subject that is not at the time of administration afflicted with the disease, disorder or condition. As used herein "treating" may also refer to preventing the recurrence of a disease, disorder or condition or of one or more symptoms associated with such disease, disorder or condition. The terms "treatment" and "therapeutically," as used herein, refer to the act of treating, as "treating" is defined above.

An "effective amount" is intended to mean that amount of an agent that is sufficient to treat, prevent, or inhibit malaria or a disease or disorder associated with malaria or another infectious disease such as a disease associated with a bacterial species. In some preferred embodiments, malaria or the disease or disorder associated with malaria is caused by a Plasmodium parasite, preferably, *P. falciparum, P. vivax, P. ovale,* or *P. malariae.*

A composition of this invention may be administered for prophylactic treatment to prevent infectious diseases caused by malarial parasites, bacteria and other infectious agents. The composition would be administered to a subject such as a human patient who is at risk for being exposed to or developing an infectious disease. Examples of this are health care workers, people who are immunocompromised, being hospitalized, travellers or anyone else who seeks preventative treatment.

The compositions of the present invention may be administered to mammals via either the oral, parenteral (such as subcutaneous, intravenous, intramuscular, intrasternal and infusion techniques), rectal, intranasal, topical or transdermal (e.g., through the use of a patch) routes. In general, the inhibitors and polyphenols are administered in

doses ranging from about 0.1 mg to about 500 mg per day, in single or divided doses (i.e., from 1 to 4 doses per day), although variations will necessarily occur depending upon the species, weight, age and condition of the subject being treated, as well as the particular route of administration chosen. However, a dosage level that is in the range of about 0.1 mg/kg to about 5 gm/kg body weight per day, preferably from about 0.1 mg/kg to about 100 mg/kg body weight per day, is most desirably employed. Nevertheless, variations may occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effects, provided that such higher dosage levels are first divided into several small doses for administration throughout the day.

The compositions may also include one or more of pharmaceutically acceptable adjuvants, carriers, diluents and excipients.

The compositions of the present invention may be administered alone or in combination with pharmaceutically acceptable carriers or diluents by either of the routes previously indicated, and such administration may be carried out in single or multiple doses. Suitable pharmaceutical carriers include solid diluents or fillers, sterile aqueous media and various non-toxic organic solvents, etc. The pharmaceutical compositions can administered in a variety of dosage forms such as tablets, capsules, lozenges, troches, hard candies, powders, sprays, creams, salves, suppositories, jellies, gels, pastes, lotions, ointments, aqueous suspensions, injectable solutions, elixirs, syrups, and the like.

The dosage forms can be prepared by methods and techniques known in the art.

The following examples are given by way of illustration of the present invention and should not be construed to limit the scope of present invention. It is to be understood that both the foregoing general description and the following detailed description are exemplary and explanatory only and are intended to provide further explanation of the invention as claimed.

## EXAMPLES

### Example 1

### Culturing of P. *falciparum*

P. falciparum strain FCK2 (CQ sensitive, IC50 18 nM) isolated from Karnataka state of India and a chloroquine resistant strain (MP-14; IC50 for chloroquine 300 nm) from the Maharashtra state of India were cultured in washed human O+ erythrocytes using the candle jar method [18]. Culture media with or without inhibitors was changed daily. Parasites were synchronized at the ring stage with 5% D-sorbitol for all and the cultures were pooled at ring or trophozoite stage with 12-15% parasitemia. Free parasites were released from the red blood cells with 0.15% saponin.

### Example 2

### Growth inhibition assay: [$^3$H] hypoxanthine incorporation

The growth of P. falciparum was quantified by measuring the incorporation of [$^3$H] hypoxanthine. Aliquots of stock solution of EGCG (as a representative of flavanoids amongst the compounds with Formulas 3-6 representing bioenhancers/inhibitors (I) or substrate analogs (A) amongst compounds with formula 1 and 2 herein exemplified by triclosan or both (I) and (A) in DMSO were placed in wells of the flat 96 well tissue culture plates under sterile conditions to final concentrations of 1pM-10 mM in 0.005% DMSO into the infected human erythrocyte suspension in culture medium. The plates were placed in candle jars and incubated at 37°C for 4, 28 or 52 h for assessing the growth at 24, 48 and 72 h respectively. At these time points, [3H] hypoxanthine (5-20 Ci/ml final concentration from a stock of 25.1 Ci/mmol (Amersham, England) was added to each well (5% v/v), incubated for an additional 20h, the cells were harvested and the radioactivity was measured. Suspensions of uninfected erythrocytes with or without 0.005% DMSO, similarly treated were used as plus and minus controls, respectively. Morphology of the parasite was microscopically examined by Giemsa-stained smears of the cultures, both before the start of [$^3$H] hypoxanthine uptake and before harvesting the cultures for [$^3$H] counts. The antimalarial activity of triclosan, flavonoids and a combination of both and their dose response curves were determined by monitoring this [$^3$H] hypoxanthine uptake as an index of the parasite growth.

Table 1: IC50 values of catechins on P. *falciparum* culture.

| Tea extracts | IC50 (μM) |
|---|---|
| EGCG | 191.0 |
| ECG | 4.7 |
| EGC | 210.9 |
| Quercetin | 20.7 |
| Buteine | 49.7 |

**Example 3**

***In vivo* antimalarial activity of triclosan in *P. berghei***

Four day suppressive test was used for checking the in vivo antimalarial activity of the substrate analog (A) with Formulas 1 and 2 more specifically among the compounds with formula 1 and 2, flavonoids with formulas 3-6 and more so EGCG alone or in combination with triclosan.

Randomly bred male Balb/c mice, 6 in numbers, weighing 22-25 grams were inoculated intravenously with 10 million *P. berghei* parasitized red blood cells. After confirming parasitemia *i. e.* on the day one of infection a single dose of either triclosan, a representative of the compounds belonging to formula 1 and EGCG among the group of flavonoids with formula 3 to 6 was injected to the mice intraperitoneally or subcutaneously twice a day for four consecutive days. Various doses of triclosan and its analogs dissolved in ethanol or dimethyl sulfoxide in 25 μl volume or EGCG in phosphate buffered saline (PBS) 100μl volume alone or in combination were injected subcutaneously once a day, for 4 days. Giemsa-stained blood smears for 5 days and thereafter was prepared from the infected animals to assess percentage parasitemia. The survival of the mice was monitored for the following week. Table 2 shows the result offered by a combination of a compound, triclosan, from within its class with formula 2 and EGCG from flavonoids (formula 3-6) survival for additional days (day 12) as compared to their untreated counterparts which succumbed to death within 6 days of infection.

Table 2: Beneficial effects of a flavonoid and triclosan combination on the survival of *P. berghei* infected mice.

| Treatment | Mice surviving from a group of 6 on Day 6 | Mice surviving from a group of 6 on Day 12 |
|---|---|---|
| 50 mM Na Phosphate buffer Ph 7.4 in 0.1M NaCl (PBS) | 0 | 0 |
| | | |
| 20 mg/kg Triclosan in 2% DMSO or Ethanol | 3 ± 1 | 1 ± 1 |
| EGCG 30 mg/kg in PBS + 20 mg/kg Triclosan in 2% DMSO or Ethanol | 6 ± 2 | 5 ± 2 |

**Example 4**

**Effect of flavonoids on the inhibitory potency of triclosan on the incorporation of [14C] acetate into fatty acids in *P. falciparum* cultures**

Fatty acid synthesis in *P. falciparum* cultures and the effect of a 2'-dihydroxyphenyl ether indicated in formula 1 and 2 with or without flavonoid was evaluated by incorporating [14C] acetate into fatty acids [Surolia and Surolia , Nature Medicine, (2001) 7, 167-173].

All flavonoids in the range of 15μM-50μM inhibited significantly (30%) and most of the 2'-dihydroxyphenyl ethers (compounds with formula 1 and 2) in the range of 2-30 μM diminished to 50-93% and triclosan at 9μM, for example, inhibited to the extent of 90% the incorporation of [$^{14}$C] acetate into fatty acids by *P. falciparum cultures.* Strikingly, however, co-incubation of flavonoids (from the group of compounds shown in formula 3, 4, 5 and 6) herein exemplified by EGCG at 40μM with 2.0μM of triclosan itself inhibited the incorporation of [$^{14}$C] acetate to 90%.

**Example 5**

**Overexpression of PfENR**

The overexpression of PfENR was carried out [22]. All buffers used contained 10 % glycerol. The purity of the enzyme was checked by SDS-PAGE. Purified enzyme was stored in -20 °C at a concentration of 5 mg/ml. Green tea catechins, crotonoyl-CoA, NADH, $NAD^+$ and all the PAGE reagents were purchased from Sigma-Aldrich, Kanamycin A and IPTG were purchased from Merck.

**Assay of PfENR**

Enzyme assays and inhibition studies were performed on a UV-Vis spectrophotometer (Jasco) at 25°C in 20 mM Tris/HCl pH 7.4 containing 150 mM NaCl [13, 14]. The standard reaction mixture of 100 μl contained 200 μM crotonoyl-CoA, 100 μM NADH and 30 nM PfENR. All the inhibitors were dissolved in DMSO. 1 % V/V DMSO was used in the standard reaction mixture. The reaction proceeds by reduction of crotonoyl-CoA to butyl-CoA with the oxidation of NADH to $NAD^+$ which is monitored at 340 nm ($\varepsilon^{M}_{340}$= 6220 M-1 cm-1).

**Example 6**

**Overexpression of EcENR**

The EcENR gene (Surolia & Surolia, Nature Medicine [2001], 7, 167-173) was cloned in pET-28a (+) expression vector between NdeI and *BamH*I restriction sites. The clone was transformed in BL21 (DE3) (Novagen) cells to express EcENR as N-terminal His-tag fusion protein. The transformed cells were grown in LB medium till 0.8 OD600 at 37°C and induced by 1 mM of isopropyl-1-thio-β-D-galactopyranoside (IPTG). The induced culture was further grown for 3 h at 37°C. Cells were collected by centrifugation (8,000 rpm, 4°C, 10 min), and stored at -20°C overnight. The cells were resuspended in 20 mM tris-HCl, 500 mM NaCl, 10%glycerol, 1 mM β-mercaptoethanol and 5 mM imadazole, pH 7.4 and lysed by sonication. The whole cell lysate was further centrifuged at 15000 rpm for 45 minute and the clear supernatant was loaded on to the Ni-NTA column. The column was washed with 40 mM imidazole in the lysis buffer and eluted with 200 mM imidazole in the same buffer. The purified proteins were stored at -20 °C.

**Assay of EcENR:**

The assay method for EcENR was carried out exactly in the same manner as discussed for PfENR.

**Example 7**

**Determination of inhibition constants of tea catechins:**

<u>Determination of IC50 values</u>

IC50 values of the tea catechins and polyphenolic compounds for PfENR were determined by plotting the percent inhibition of PfENR at various concentrations of inhibitory compounds. In the standard reaction mixture mentioned in Example 1 various concentrations of EGCG or other catechins were added and the percent inhibition was calculated from the residual enzymatic activity. The percent activity thus calculated was plotted against log of concentration of respective catechins. The data was analyzed by nonlinear regression method using sigmoidal model and IC50 values for each catechin was calculated from the sigmoidal curve.

Various concentrations of EGCG (10 nM to 2 μM) were used to determine the activity of PfENR. The percent inhibition was calculated from the residual PfENR activity and was plotted against log [EGCG] to obtain IC50 value. The sigmoidal curve indicates the best fit for the data. (Figure 1 (A))

Three of the tea catechins (EGCG, EGC, ECG) and two polyphenols (Quercetin and butein), which produced IC50 values in the range of 5 to 30 μM for the *E. coli* ENR[7], were selected for the present study. Amongst the five compounds tested EGCG was the most potent (IC50 = 250 ± 6.2 nM) inhibitor of PfENR (Figure 1A). The IC50 values of other compounds are shown in Table 1. Among the two polyphenols tested quercetin gave the IC50 value of 2.5 ± 0.078 μM and butein 12.5± 0.67μM. In comparison to the reported data on the *E. coli* counterpart [7] all these compounds showed improved IC50 values (Table 3).

Table 3: Comparison of IC50 values of different selected catechins on PfENR and EcENR [7]. The lowest IC50 value (250nm) for PfENR was obtained with EGCG.

| Flavonoid | IC50 [μM] with PfENR | IC50 [μM] values with EcENR |
|---|---|---|
| EGCG | 0.250 ± 0.0062 | 15 |
| ECG | 0.500 ± 0.016 | 10 |
| EGC | 7.0 ± 0.085 | >100 |
| Quercetin | 2.5 ± 0.078 | 20 |
| Buteine | 12.5 ± 0.67 | 30 |

Calculation of dissociation constants (Ki)

Ki of individual catechins with respect to NADH and crotonoyl-CoA were determined in separate experiments. The data for NADH was collected at three fixed concentrations of NADH (50μM, 100μM and 200 μM) while varying the catechin concentration from 1 nM to their respective IC50 values and keeping crotonoyl-CoA concentration fixed at 200 μM. The data for crotonoyl-CoA, was collected at two fixed concentrations of crotonoyl-CoA (100 μM and 200μM) and catechin concentration was varied from 1 nM to the IC50 value, while NADH concentration was fixed at 100 μM. All the data were analyzed by Dixon plot [8].

Kinetic parameters for all the five catechins were determined individually against cofactor NADH and the substrate analog crotonoyl-CoA, respectively. Earlier, EGCG has shown competitive kinetics against NADH with EcENR [7]. Like wise in the case of PfENR, all the five compounds showed competitive kinetics with NADH (Figure 1B). Figure1B shows the inhibition kinetics of EGCG with respect to NADH. 30 nM PfENR was assayed in presence of 200 μM crotnoyl CoA, 250 nM EGCG and 50 μM [•], 100 μM [■] and 200 μM [▲] of NADH. The cumulative effect of NADH and EGCG on PfENR inhibition was determined using Dixon plot. Ki of EGCG was calculated from the X-intercept using equation for competitive kinetics. Their Ki values are shown in Table 4. With respect to the substrate analog, crotonoyl-CoA, these compounds followed uncompetitive kinetics (Figure 1C). Figure 1C shows the inhibition kinetics of EGCG with respect to crotonoyl CoA. The enzyme PfENR was assayed at two fixed concentration 100 μM [•] and 200 μM [■] of crotonoyl CoA in presence of 250 nM of EGCG and 100 μM of NADH. Assuming uncompetitive kinetics Ki was calculated from the X-intercept of Dixon plot. The Ki values of the individual compounds were calculated from the Dixon plot. EGCG was the best compound with Ki value of 79.0 ± 2.67 nM and the least effective compound was buteine with Ki value of 2.97 ± 0.077 μM against crotonoyl-CoA. For the other catechins, Ki values against crotonoyl-CoA are given in Table 4. Analysis of the kinetic data clearly shows that EGCG along with the other catechins competes with NADH for binding to PfENR and inhibits its activity by preventing the binding of NADH. On the other hand uncompetitive kinetics with respect to crotonoyl-CoA shows that crotonoyl-CoA somehow facilitates the binding of catechins. Notably, such a potentiation of the interactions of catechins by the substrate for other enzymes has not been observed earlier.

**Example 8**

**Potency assay**

The effect of catechins on triclosan binding was judged by the potency assay and these assays for triclosan inhibition of PfENR were designed as described earlier [14]. The effect of addition of tea catechins (IC50 values) without pre-incubation and with 30 minutes pre-incubation with triclosan (150 nM) on the activity of PfENR (30 nM) were studied. The formation of $NAD^+$ was monitored at 340 nm after the addition of 200 μM crotonoyl-CoA and 100 μM NADH. Control reaction was set up without the addition of triclosan with tea catechins and 1% DMSO. The accumulation of the product [$NAD^+$] in each case was plotted against time (Figure 2). The potency assay shows the slow onset of inhibition by triclosan. In Figure 2 Curve "a" is a control reaction without EGCG and triclosan, curve "b" is an inhibition reaction in presence of 150 nM triclosan, curve "c" depicts the onset of inhibition in presence of 250 nM EGCG and 150 nM triclosan without preincubation and curve "d" shows further potentiation of inhibition when EGCG and triclosan were preincubated with PfENR for 30 minutes.

Binding of triclosan to both PfENR and EcENR is potentiated by $NAD^+$ [14]. The competitive kinetics of tea catechins with PfENR with respect to NADH tempted us to investigate whether these compounds can mimic the effect of $NAD^+$ and potentiate triclosan binding to PfENR. From Figure 2, it is clear that the control reaction showed the linear mode of $NAD^+$ accumulation with respect to time (curve "a"), addition of 150 nM triclosan to the reaction mixture impeded

the rate of the reaction in a gradual manner (curve "b"). The onset of inhibition was faster when tea catechin (EGCG) was added along with triclosan (curve "c"). Incubation of EGCG and triclosan for 30 minutes with PfENR further hastened the onset of inhibition (curve 'd"). Thus, the potency assay indicated that EGCG promoted the binding of triclosan to PfENR. It also hinted that triclosan behaved as a slow tight binding inhibitor in the presence of EGCG.

**Determination of Ki of Triclosan in presence of the tea catechins**

For calculating Ki the triclosan concentration was varied from 0 to 700 nM at two different catechin concentrations (around IC50 value of individual catechins). Likewise, the Ki of each catechin was determined at two fixed concentration of triclosan (50 nM and 100 nM) while varying the concentration of the individual catechins. All the data of individual experiments were repeated thrice and the mean value was taken for data analysis. The Ki values were determined for each experiment from the X-intercept of the Dixon plot [8].

In the presence of NAD+, Ki of 53 nM for triclosan with PfENR was calculated by Dixon plot [14]. In the presence of 200 nM of EGCG the Ki of triclosan came down to 1.0 ± 0.087 nM and followed uncompetitive kinetics (Figure 3A). Inhibition assay of 150 nM triclosan in presence of 50 nM [•] and 100 nM [■] of EGCG was conducted to assess EGCG effect on triclosan. As seen in the figure triclosan followed uncompetitive kinetics with EGCG. Ki of triclosan was calculated to be 1nM from the x-intercept. All the compounds tested potentiated the binding of triclosan to PfENR (Table 1 and Figure 3).

Table 4 : Comparison of detail kinetic parameters of the tea catechins by spectrophotometric [Dixon plot] and fluorescence titration studies.

| Tea catechins | Ki w.r.t.* NADH [μM] | Ki w.r.t. crotonoyl-CoA [μM] | Ki of TCL [nM] | Ki in presence of TCL [nM] | Ki of TCL by fluorescence quenching [nM] |
|---|---|---|---|---|---|
| EGCG | 0.186 ± 0.00798 | 0.079 ± 0.00267 | 1.0 ± 0.087 | 8.0 ± 0.56 | 7.29 ± 1.02 |
| ECG | 0.291 ± 0.0076 | 0.102 ± 0.0046 | 8.0 ± 0.084 | 16.0 ± 0.45 | 28.31 ± 5.74 |
| EGC | 3.75 ± 0.054 | 2.0 ± 0.078 | 8.25 ± 0.081 | 17.56 ± 0.67 | 52.71 ± 8.61 |
| Quercetin | 1.09 ± 0.089 | 0.473 ± 0.011 | 10 ± 0.069 | 22.0 ± 0.85 | 30.54 ± 7.62 |
| Butein | 5.5 ± 0.1 | 2.97 ± 0.077 | 13.72 ± 0.42 | 14.0± 0.57 | 71.02 ± 9.67 |

To test whether triclosan could potentiate inhibition of PfENR by tea catechins Ki for each catechin was determined in the presence of triclosan. In this case uncompetitive kinetics was observed (Figure 3B). Figure 3B shows the effect of triclosan on EGCG inhibition. EGCG gave uncompetitive kinetics with respect to 50 nM [•] and 100 nM [■] of triclosan as can be seen from the Dixon plot. Ki of EGCG was calculated to be 8.0 ± 0.56 nM in presence of triclosan.

The data showed a marked improvement in the Ki values of each inhibitor. As a model compound, the Ki of EGCG was improved by a factor of 10 and was calculated to be 8.0 ± 0.56 nM in the presence of triclosan whereas it was 79.0 ± 2.67 nM in the absence of triclosan. For the rest of the compounds also, the potencies were enhanced by triclosan (Table 1).

**Example 9**

**Analysis of [3H] EGCG binding to PfENR:**

Binding studies of [$^{3}$H] EGCG were carried out with PfENR in the absence and presence of triclosan by using gel filtration chromatography and filter binding assay. Binding studies were carried out at 25 °C for 20 minutes in 20 mM Tris, pH 7.4 containing 150 mM NaCl 1 μM [3H]EGCG (specific activity 5 Ci/mmol), 1 % v/v DMSO and 8 μg of PfENR in 50 μl of reaction volume, with varying concentration of triclosan. Briefly, for gel filtration chromatography, 2 ml Sephadex G-25 column was packed and equilibrated with the reaction buffer. For each run 50 μl of the reaction mixture was loaded and 20 fractions of 100 μl were collected at 0.25 ml/min flow rate. All the fractions were spotted on Whatman 3 filter paper and dried before taking the counts. In the filter binding assay method, the reaction mixtures were directly applied on the activated Polyvinylidene difluoride (PVDF) membrane fixed in a filtration assembly. The filters were washed with 200 μl of reaction buffer and dried. Dried filters were transferred to vials containing 5 ml scintillation fluid and the radioactivity measured using a Hewlett Packard liquid scintillation counter.

To check the binding of [$^3$H] EGCG alone, five concentrations of [$^3$H] EGCG (0.1 to 10 μM), as indicated in Figure 4a, were taken. The concentration of [$^3$H] EGCG (bound) was plotted against [$^3$H] EGCG (free) to get the binding constant. To determine the binding constant of EGCG in the presence of triclosan, the above experiments were repeated in the presence of 10 μM triclosan (Figure 4C).

Finally, to determine the binding constant of triclosan in the presence of EGCG, various concentrations of triclosan were used (25 nM to 1 μM as indicated in Figure 4d) with 1 μM [3H]EGCG. [$^3$H] EGCG showed saturation binding curve in the presence of increasing concentration of TCL and a binding constant of 451 ± 7.7 nM was obtained. (Figure 4d) Double reciprocal plot of the same data is shown in the inset.

The binding constants for the above experiments were determined from saturation plots and by Scatchard plot or double reciprocal plot using equation 2a and 2b described under evaluation section.

Binding of [$^3$H]EGCG to PfENR was analyzed by filter binding assay and followed saturation kinetics with dissociation constant of 438 ± 8.2 nM, where [$^3$H]EGCGf and [$^3$H]EGCGb are the bound and free concentration of [$^3$H] EGCG, respectively. The Scatchard plot of the data is shown in the inset. The binding of [$^3$H] EGCG to PfENR followed saturation kinetics (Figure 4A). The dissociation constant was calculated by saturation kinetics [equation 2a] as well as by Scatchard plot (Equation 2b) (Figure 4a inset), which gave the values of 438 ± 8.2 nM and 512 ± 28.78 nM, respectively. We then calculated the dissociation constant of EGCG in the presence of 10 μM triclosan. Figure 4B shows the gel filtration profile of [$^3$H] EGCG binding to PfENR. 2 ml Sephadex G-25 column having a void volume [Vo] of 680 μl was used for gel filtration experiments. Gel filtration profile is shown as plot of the fraction numbers versus [$^3$H] CPM. As can be seen [$^3$H] CPM in the presence of 10μM triclosan (TCL) [▲] were dramatically increased near the void volume.

As can be seen from the gel filtration profile (Figure 4B) there is a noticeable increase in the counts of bound [$^3$H] EGCG to PfENR in the presence of triclosan. The Kd calculated by saturation kinetics (Figure 4C) and Scatchard plot (inset, Figure 4C) was 89 ± 2.3 nM and 77.6 ± 5.3 nM, respectively. Finally, binding constant of triclosan was determined at fixed concentration of [$^3$H] EGCG (1 μM) and variable triclosan concentration which also followed saturation kinetics [$^3$H] EGCG showed saturation binding curve in the presence of increasing concentration of TCL and a binding constant of 451 ± 7.7 nM was obtained. Double reciprocal plot of the same data is shown in the inset.

(Figure 4D). Association constant of triclosan was determined to be 22.18 ± 1.7 $\mu M^{-1}$ by saturation kinetics and 14 ± 0.77 $M^{-1}$ by double reciprocal plot (inset of Figure 4D). The dissociation constants were 45.1 ± 8 nM and 71 ± 4.3 nM, respectively. From the above radiolabeled binding data it became evident that the presence of triclosan promoted the binding of [$^3$H] EGCG to PfENR. Figure 4c shows the binding of [$^3$H] EGCG in the presence of Triclosan. Binding was assessed using PVDF membranes. Again a saturation behavior was observed in the presence of 10 μM TCL, [$^3$H] EGCG. A non-linear least squares fit of the data yielded a Ki value of 89 ± 2.3 nM. A similar Ki value was obtained from the Scatchard plot that has been shown in the inset. Figure 4D shows the influence of Triclosan on [$^3$H] EGCG binding. [$^3$H]EGCG showed saturation binding curve in the presence of increasing concentration of TCL and a binding constant of 451 ± 7.7 nM was obtained. Double reciprocal plot of the same data is shown in the inset. In all the above cases saturation curves were plotted using equation 2a and Scatchard plot by using equation 2b.

### Example 10

### Determination of association or kon and dissociation or koff rate constants of triclosan in the presence of catechins

The association rate constants of triclosan with PfENR in the presence of different tea catechins were determined by monitoring the onset of inhibition in the enzyme reactions, which contained 100 μM NADH, 200 μM crotonoyl-CoA, individual catechins at their respective IC50 values and various concentrations of triclosan (0 to 700 nM). The reaction was started by the addition of 50 nM of PfENR. For each concentration of triclosan the formation of $NAD^+$ was plotted against time by non-linear regression method using equation 3 discussed below.

The dissociation rate constants for triclosan for various tea catechins-PfENR complexes were determined by dilution method. PfENR, 15 μM was incubated with 15 μM triclosan and 10 μM of individual tea catechins for 30 minutes. The reaction was started with 1000 fold dilution of the PfENR-triclosan and EGCG ternary complex with buffer containing competing NADH and crotonoyl-CoA to a final concentration of 100 μM and 250 μM, respectively. The reaction was monitored at 340 nm and the formation of ($NAD^+$) was plotted as a function of time using equation 3 to get the dissociation constant by non-linear regression analysis of the data.

The dissociation constant koff or k6 was calculated directly by plotting the NAD+ formed against time in equation 3a using non-linear least-squares fit. Keeping EGCG as a model compound, the koff or k6 of triclosan in the presence of EGCG was calculated to be $1.6\times10^{-5} \pm 0.078\times10^{-5}$ $s^{-1}$. For all the other catechins the $k_{off}$ data are summarized in Table 4. The regain of activity after dilution indicates that triclosan and tea catechins do not make a covalent adduct with PfENR (Figure 7). The very slow dissociation constant ($k_6$) reveals that the ternary complex [EI*] is highly stable and accounts for the extremely potent inhibition by triclosan in the presence of tea catechins. PfENR was incubated with

equimolar quantity of triclosan and 10 μM of EGCG for 30 minutes. The reaction mixture was diluted 1000 times with competing NADH and crotonoyl-CoA. Oxidation of NADH to $NAD^+$ was plotted against time. The data were analyzed by non-linear regression using equation 3. $k_6$ of triclosan thus calculated was $1.6\times10^{-5}$ $s^{-1}$.

Table 5 : Determination of the overall inhibition constant of triclosan in the presence of tea catechins using steady state kinetics. Rate constants, $k_5$, $k_6$ and K* values were determined using equations 3, 4 and 5, respectively

| Tea extracts | Association rate constant [$k_5$] of $k_6$ of triclosan, [$10^{-3}$] $S^{-1}$ | Dissociation rate constant [$k_6$] of $k_5$ of TCL, [$10^{-5}$] $S^{-1}$ | Overall inhibition constant for TCL [Ki*] [pM] |
|---|---|---|---|
| EGCG | 8.4 ± 0.24 | 1.6 ± 0.078 | 1.9 ± 0.46 |
| EGC | 5.4 ± 0.097 | 7.4 ± 0.68 | 109 ± 8.6 |
| ECG | 6.3 ± 0.135 | 4.1 ± 0.27 | 52.06 ± 3.56 |
| Quercetin | 3.1 ± 0.0786 | 8.7 ± 0.334 | 280.6 ± 11.78 |
| Buteine | 2.1 ± 0.108 | 13.0 ± 0.809 | 849.33 ± 64.47 |

Association rate constant ($k_5$) or onset of inhibition of triclosan at fixed concentration of tea catechins were calculated from the progress curves generated after fitting the data to equation 3a. Progress curves gave the value of $k_{obs}$ and the $k_5$ or association rate constants were determined by fitting the values of kobs with experimentally determined $k_6$ values in equation 4. The values of $k_5$ against each tea compound are given in Table 5. Low value of $k_5$ indicates the slow isomerization of the weak ternary complex into more stable ternary complex of PfENR-TCL-EGCG.

The overall inhibition constant (Ki*) of triclosan with each of the catechins and polyphenols was calculated by equation 5 using the respective Ki values (Table 5) Ki, k5 and k6 of triclosan with respect to individual tea extracts. Out of the five compounds tested, triclosan gave the best Ki* value of 1.9 ± 0.46 pM with EGCG. The overall inhibition constant (Ki*) of triclosan in the presence of EGCG is roughly 50 times better than in the presence of NAD+ where it is 96 pM [14].

**Evaluation of the kinetic data:**

Initial rate constants were determined using Dixon plot [8] assuming the reaction to be competitive for tea catechins against NADH and uncompetitive for tea catechins against triclosan. The data were plotted using the following Dixon equations for competitive and uncompetitive kinetics, respectively.

$$\frac{1}{v} = \frac{K_m \times [I]}{V_{max} \times K_i \times [S]} + \frac{1}{V_{max}(1 + \frac{K_m}{[S]})} \quad (1a)$$

$$\frac{1}{v} = \frac{[I]}{V_{max} \times K_i} + \frac{1}{V_{max}(1 + \frac{K_m}{[S]})} \quad (1b)$$

where Km is the Michaelis constant, Vmax is the maximal catalytic rate at saturating substrate concentration [S], [I] is the concentration of inhibitor and Ki is the dissociation constant of the inhibitor. (Equation 1a. is for competitive kinetics and 1b is for uncompetitive kinetics).

The binding of [3H] EGCG to PfENR was analyzed by saturation and Scatchard plot [9] using the equations 2a and 2b, respectively.

$$[S]_b = \frac{[S]_{b\max} \times [S]_f}{K_d + [S]_f} \qquad (2a)$$

Where [S]b is the bound, [S]f is free and [S]bmax is the maximum binding of the ligand being analyzed and Kd is the dissociation constant.

$$\frac{[S]_b}{[S]_f} = -\frac{1}{K_d[S]_b} + \frac{n[E]_t}{K_d} \qquad (2b)$$

Where, n is the number of identical and independent ligand binding sites per molecule of enzyme and [E]t is the total concentration of enzyme.

Time dependent reversible inhibition can be described by any of the two mechanisms described below [10, 11]. In mechanism 1, the inhibitor and enzyme react with each other in a single step bimolecular reaction to form an effective enzyme-inhibitor complex. This kinetics results either from slow association or sometimes slow dissociation of the inhibitor.

$$E + I \underset{k_4}{\overset{k_3}{\leftrightarrow}} E.I \qquad \text{(Mechanism 1)}$$

In mechanism 2, the inhibition takes place in two steps, in the first step the enzyme binds rapidly with the inhibitor forming EI complex which, then slowly isomerizes to a more stable and effective EI* complex

$$E + I \underset{k_4}{\overset{k_3}{\leftrightarrow}} E.I \underset{k_5}{\overset{k_6}{\leftrightarrow}} E.I^* \qquad \text{(Mechanism 2)}$$

In both the mechanisms it is presumed that the slow binding inhibition step is reversible.

The biphasic progress curves which is typical for slow tight binding inhibition were fitted to equation 3 [10, 11, 12, 14] using non-linear least squares fitting procedure.

$$P = v_s \times t + \frac{(v_o - v_s)[1 - \exp(-k_{obs} \times t)]}{k_{obs}} + C \qquad (3a)$$

Where P is the concentration of the product formed at any given time t, vo is the initial velocity, vs is the final steady-state velocity, $k_{obs}$ is the apparent first order rate constant for the establishment of the final steady-state equilibrium and C is a constant, non-zero Y-intercept term [12]. Corrections were made for the variation of the steady-state velocity with the inhibitor concentrations using equation 3b and 3c as described earlier by Morrison and Walsh [10]

$$v_s = \frac{k_7 SQ}{2(K_m + S)} \qquad (3b)$$

$$Q = [(Ki' + It - Et) + 4Ki'Et]1/2 - (Ki' + It - Et) \quad (3c)$$

Where Ki' = Ki*(1 + S/Km), It and Et are the total inhibitor and enzyme concentrations, respectively.

k7 is the rate constant of the product formation $[E + S \underset{k_2}{\overset{k_1}{\leftrightarrow}} ES \overset{k_7}{\rightarrow} E + P]$

The relationship between Ki, $k_5$, $k_6$ and $k_{obs}$ is described by equation 4.

$$k_{obs} = k_6 + k_5\left(\frac{\frac{1}{K_i}}{1+\left(\frac{[S]}{K_m}\right)+\left(\frac{[I]}{K_i}\right)}\right) \quad (4)$$

The overall inhibition constant Ki* is calculated by equation 5.

$$K_i^{*} = K_i \times \left(\frac{k_6}{(k_5 + k_6)}\right) \quad (5)$$

Examination of the progress curves of inhibition by triclosan in the presence of different catechins yielded a similar pattern. The control reaction was set up with individual catechins without triclosan and the rest of the reactions contained triclosan from 0 to 700 nM. From the progress curves (Figure 5), it can be stated that for each concentration of triclosan the initial and steady-state velocities decrease exponentially with time and at higher triclosan concentrations the steady-state reaches rapidly but with a decrease in steady-state velocity (vs). This implies that in the presence of tea catechins, triclosan interacts with the PfENR-catechin complex rapidly to form an initial complex and then slowly converts into a stronger PfENR-catechin-triclosan complex (mechanism 2).

For each concentration of triclosan progress curve was generated using equation 3a. In the standard reaction mixture, 100 nM of EGCG was added which served as the control reaction. As indicated in the figure triclosan was added in the control reaction at various concentrations [0-700 nM].

The individual progress curves were analyzed by equation 3a, b, c from which a series of $k_{obs}$ values were determined for each concentration of triclosan for each tea catechin. The determined $k_{obs}$ values were plotted against respective triclosan concentrations which resulted in a hyperbolic curve (Figure 6). The hyperbolic curve is diagnostic of a two phase binding behavior of the inhibitor [10, 11] and thus proves that triclosan followed biphasic nature of binding reflecting slow, tight binding behavior with PfENR in the presence of EGCG and other catechins. The first phase consists of rapid formation of weak ternary complex of PfENR-TCI-EGCG [EI, in mechanism 2] which slowly isomerizes into a more stable ternary complex [EI* in mechanism 2]

The initial rate constant ($k_{obs}$) for each triclosan concentration added was calculated from the progress curves using equation 3a. The $k_{obs}$ thus calculated and the respective Triclosan concentrations was fitted to equation 4 and the best fit gave hyperbolic curve demonstrate two phase inhibition mechanism indicating that triclosan is behaving as a slow-tight binding inhibitor in the presence of EGCG.

**Example 11**

**Fluorescence analysis of triclosan binding in the presence of tea catechins:**

Fluorescence analysis of triclosan binding was done as mentioned elsewhere [14] using JobinYvon Horiba fluorimeter. The excitation and emission monochromator slit widths were adjusted at 3 nm. All the reactions were performed in 3 ml quartz cuvette with constant stirring at 25 °C. For the binding studies, 4 μM PfENR was incubated with

different tea compounds at their saturating concentrations in 20 mM tris and 150 mM NaCl, pH 7.4, excited at 295 nm and change in the fluorescence intensity at the emission maximum of PfENR (340 nm) was recorded. PfENR-catechin complex were titrated with increasing concentrations of triclosan from 0 to 30 μM. The difference in fluorescence intensity upon triclosan binding was analyzed using the following equations [13] to calculate Ki value of triclosan.

$$F_o - F = \frac{\Delta F_{max}}{1+\left(\frac{K_i}{[I]}\right)} \quad (6)$$

Where, Fo-F is the rapid fluorescence change, ΔFmax is maximum change in fluorescence, Ki is dissociation constant and [I] is inhibitor concentration.

The onset of inhibition was calculated from the time course of fluorescence quenching at 340 nm after adding triclosan to PfRNR-catechin solution for 20 minutes. For tight binding inhibitors k6 can be considered negligible at the early onset of inhibition. So, k5 can be directly calculated from the following equation [13],

$$k_{obs} = \frac{k_5[I]}{\{K_i + [I]\}} \quad (7)$$

Where, $k_{obs}$ is the rate constant for the loss of fluorescence.

The inner filter effect was corrected by using the following equation [14],

$$F_c = F anti\log[(A_{ex} + A_{em})/2] \quad (8)$$

Where, Fc is the corrected fluorescence and F is the measured one, $A_{ex}$ and $A_{em}$ are the absorbance of the reaction solution at the excitation and emission wavelengths, respectively.

The triclosan binding to PfENR-catechin complex was analyzed by monitoring the rapid fluorescence decrease (Fo-F), at 340 nm after each addition of triclosan (Figure 8). 5 μM PfENR preincubated with 100 nM EGCG was used in this study. Triclosan was added sequentially from 0 to 30 μM. The samples were excited at 290 nm and the emission was recorded at 340 nm. Change in fluorescence [Fo-F], after correcting for inner filter effect was plotted against triclosan concentrations by using equation 6. The best fit of the data yielded a hyperbolic curve.

The Ki of triclosan against each of the five catechins were calculated after fitting the data to equation 6. Before calculating Ki and k5 from fluorescence data, the corrections for inner filter effect were made using equation 8. In the presence of EGCG, triclosan gave Ki value of 7.29 ± 1.02 nM, Ki for the rest of the compounds are shown in Table 3. For calculating $k_5$, time course of fluorescence quenching for 30 minutes was monitored resulting in rapid fluorescence decrease for the initial phase followed by a slow decrease which proves the binding to be a two step mechanism (Figure 9). The initial rapid fluorescence decrease depicts the formation of the reversible PfENR-catechin-triclosan ternary complex and the second phase is the slow conversion to the tight ternary complex. $k_5$ of triclosan in presence of EGCG calculated from the slow decrease of fluorescence using equation 7 was $0.0072 \pm 0.84 \times 10^{-5}$ $s^{-1}$. The data obtained from fluorescence studies are in agreement with the enzyme inhibition kinetics data.

5 μM PfENR was incubated with 5 μM of EGCG served as a control reaction. The time course of fluorescence quenching was followed for 20 minutes. The excitation and emission wavelengths were set at 295 nm and 340 nm, respectively. 20 μM triclosan was added to the control reaction and changes in fluorescence were monitored for 20 minutes. A rapid fall in fluorescence intensity suggests the quick formation of PfENR-EGCG-Triclosan ternary complex, which undergoes slow transition to the more stable ternary complex.

**Example 12**

**Docking of inhibitors with P. falciparum and *E. coli* FabIs:**

All the docking simulations were done using AutoDock 3.05 [15] and MOE [Molecular Operating Environment] [16].

Preparation of the Receptor and Ligand Molecules: The crystal structures of PfENR (PDB Code: 1NHG) and EcFabI (PDB Code: 1QSG) submitted to PDB (www.rcsb.org) by Perozzo et. al. [17], and Stewart et. al. [18] and *Pidugu et. al.* [16], respectively, were used for docking studies. The structure co-ordinates were converted into mol2 format with MMFF94 charges assigned using the MOE [Molecular Operating Environment] suite of programs [16]. The molto2pdbqs utility (provided with AutoDock program) was used to prepare the input receptor file containing fragmental volume and solvation parameters. Inhibitors were also built using MOE and energy minimized with MMFF94 charges. The AutoTors utility [provided with AutoDock program] was used to define torsion angles in the ligands prior to docking.

Docking Simulations: Grid maps for docking simulations were generated with 80 grid points (with 0.375 Å spacing) in x, y and z directions centered in the active site using the AutoGrid utility of AutoDock program. Lennard-Jones parameters 12-10 and 12-6 (supplied with the program package) were used for modeling H-bonds and van der Waals interactions, respectively. The distance-dependent dielectric permittivity of Mehler and Solmajer [19] was used in the calculations of the electrostatic grid maps. The Lamarckian genetic algorithm (LGA) with the pseudo-Solis and Wets modification (LGA/pSW) method was used with default parameters except the "maximum number of energy evaluations" which was increased to 2.5 million from 250 thousand. Hundred independent runs were conducted for each inhibitor.

Modeling of the ternary complex: The ternary complex of PfENR-EGCG-TCL was achieved by first docking the tea catechins with PfENR to generate the binary complex and then docking triclosan to this binary complex. Briefly, the top ranked conformation of the biggest cluster in the set of 100 runs of EGCG docking with PfENR was chosen. Now, this binary complex was prepared as receptor (as described above) and triclosan was docked using AutoDock with this binary complex, using the same protocol as mentioned above. Since the receptor remains rigid during the docking simulation by AutoDock, the docked complexes were energy-minimized to 0.1 kcal/mol keeping the receptor and the ligand flexible. This accounts for the minor structural changes that take place in the receptor as well as in the ligand. The ligand-receptor interactions were calculated using LPC/CSU Server [http://ligin.weizmann.ac.il/cgi-bin/lpccsu/LpcCsu.cgi].

Docking studies with AutoDock show that tea catechins indeed occupy NAD+ binding site (Figure 10A). Docking energies for all the five catechins are summarized in Table 6. EGCG is represented as ball and stick model and the enzyme in ribbons. PfENR is represented as ribbon (1) with corresponding EGCG as (2), EcENR is represented as ribbon (3) with corresponding EGCG as (4). The figure was generated using Weblab Viewerlite and rendered using POV-Ray.

Table 6: Summary of mean docking energies of the catechins as determined from their individual binary complexes with PfENR

| Tea extracts | Mean Docked Energy [Kcal/mol] | IC50 values with PfENR |
|---|---|---|
| EGCG | -18.00 | 0.250 ± 0.062 |
| ECG | -15.27 | 0.500 ± 0.016 |
| EGC | -12.31 | 7.0 ± 0.085 |
| Quercetin | -12.01 | 12.5 ± 0.67 |
| Buteine | -11.46 | 2.5 ± 0.078 |

The main reason for the high affinities of these compounds for ENRs is the extensive hydrogen bonding network involving their hydroxyl groups. Overall, the number of favorable interactions is more in the case of catechin-PfENR complex compared to catechin-EcENR complex accounting for the higher affinity of catechins for PfENR (Table 7), As a model case we compared the interactions of EGCG with PfENR and EcENR. The galloyl moiety plays a major role in the affinity of EGCG with both ENRs. It occupies the same pocket as adenine ring of NADH in the cofactor binding site of the enzyme. Its phenol ring makes aromatic stacking interaction with the side-chain Trp35 of PfENR similar to the adenine ring of NADH. It is also involved in hydrophobic interactions with Leu120. In case of EcENR the galloyl moiety makes T-shaped aromatic interactions with Phe93.

Table 7 : Comparison of all the stabilizing and destabilizing contacts made by EGCG in binary complexes with PfENR and EcENR.

| Contacts made in binary complexes | Hydrogen Bonds | Hydrophobic Contacts | Aromatic-Aromatic Contacts | Hydrophilic-Hydrophobic Contacts [Destabilizing] | Acceptor-Acceptor [Destabilizing] |
|---|---|---|---|---|---|
| ECGC-PfENR | 27 | 21 | 10 | 19 | 2 |
| ECGC-EcFabl | 18 | 34 | 6 | 24 | 2 |

Benzopyran moiety of EGCG occupies a cavity which otherwise accommodates the phosphate group of the adenine half of NADH in both PfENR and EcENR. The hydrogen bonds are comparatively longer in case of EcENR than those in case of PfENR and hence weaker. Also, benzopyran moiety contributes most of the unfavorable contacts with the two enzymes and the number of such contacts is more in case of EcENR compared to PfENR as calculated from the LPC-CSU server. There is a striking difference in the interactions of non-galloyl phenol ring with PfENR and EcENR. In the former it occupies the same space as occupied by the ribose and phosphate groups of the nicotine moiety of NADH, but in EcENR it occupies the space which accommodates ribose of the adenine moiety of NADH, a flip of 180 degrees, and it further extends in this pocket to make more interactions with the enzyme.

Overall, the favorable interactions between EGCG and ENR are more and unfavorable contacts significantly less in case of P. *falciparum* while for EcENR the situation is reverse (Table 7). This explains the higher affinity of EGCG for PfENR compared to EcENR.

Since EGCG is smaller than NADH in length and mimics the adenine moiety better, it occupies the adenine binding pocket. The nicotinamide binding pocket is now partially filled by triclosan. We know that stacking of the aromatic rings of triclosan and $NAD^+$ in the ternary complex of PfENR-$NAD^+$-TCL play a major role in the binding of triclosan [15, 17], similar stacking interactions also take place between the dichloro-phenyl ring of triclosan and non-galloyl phenyl ring of EGCG leading to a strong PfENR-EGCG-triclosan ternary complex (Figure 10B). The ortho-positioned chlorine has hydrophobic interactions with the surrounding Lys189, Tyr181, Thr170 and Leu169. The para-positioned chlorine has hydrophobic interactions with Met185, Ala223, lie227 and Asn122. The OH on the phenol ring is within hydrogen bonding distance with O of Ala216 and Gly217. The chlorine atom on this ring has hydrophobic interactions with Tyr171, Ala236, Pro218 and Phe232 and the benzene ring has aromatic interactions with Tyr171, Tyr15, Tyr181 and hydrophobic interactions with lie233, Pro218 and Tyr171 (Figure 10B). From the above studies it becomes apparent that the ternary complex of PfENR-EGCG-TCL is very strong and is stabilized by numerous hydrophobic and hydrogen bonds and mimics the PfENR-$NAD^+$-TCL ternary complex.

Figure 10B represents the EGCG and triclosan bound with PfENR in a modeled ternary complex. Amino acid residues are represented in lines, and EGCG and triclosan are in ball and stick. Atom colors are shown as; C in grey, O in red, N in blue, Cl in green and S in yellow. H bonds are depicted as green dotted lines. Hydrogens are not shown for sake of clarity. Amino acids from the pdb structure with code 1 NHG have been renumbered continuously from 1 to 289 for first monomer. Docking studies were performed using Autodock program as detailed in methods.

**Example 13**

**Cloning, over expression and purification of recombinant human aldose reductase (ALR2):**

The cloning of human ALR2 gene was done as described before [20] with slight modification. In brief, the open reading frame of the human AR2 gene (Accession GenBank/EMBL Data Bank number J05017) was amplified by the polymerase chain reaction from its cDNA and cloned into the T7 RNA polymerase-based vector pET28a(+) vector (Novagen). The positive clone was sequenced and then transformed into BL-21(DE3) cells for expression. The culture (5 L) was grown at 37 °C till OD600 of 0.8, induced by 1 mM IPTG and grown further at 37 °C for next 3h. The cells were pelleted, sonicated and the clear supernatant was loaded to Ni-NTA resin. The purified hexa-His tagged human AR2 was subjected to thrombin cleavage and loaded on topurified from Ni-NTA column and the tag was removed by thrombin cleavage. The His-tag cleaved AR2 was further loaded on DEAE Sephadex A-50 column (Pharmacia) and eluted with an NaCl gradient. The purity of the final enzyme preparation was checked on 12 % SDS-PAGE. The purified enzyme showed single band of - 36 kDa on SDS-PAGE and showed similar activity as described earlier [21]

**Example 14**

**Determination of dissociation constants (Ki) of glycerladehyde in the absence and presence of quercetin and vice versa with aldose reductase**

The dissociation constants for binary complexes (aldose reductase-glyceraldehyde or aldose reductase-quercetin) and ternary complexes (aldose reductase-quercetin-glycerladehyde or aldose reductase-glyceraldehyde-quercetin) were determined by fluorescence quenching experiments as described in Example 11. For aldose reductase the excitation and emission wavelengths were 288 nm and 342 nm, respectively. Here, 1 μM of aldose reductase was titrated with increasing concentration of glyceraldehydes (10 nM to 1 mM) and in the case of quercetin (100 pM to 5 μM) in 100 mM potassium phosphate buffer (pH 7.0) at 25 °C in a 3 ml cuvette with constant stirring. To determine the Ki of glyceraldehydes in the presence of quercetin, 2 μM of quercetin was preincubated with 1 μM of aldose reductase in the cuvette of 15 minutes and then titrated with glyceraldehyde concentrations mentioned above. Similarly, for determining the Ki of quercetin in the presence of glyceraldehyde, aldose reductase was pre incubated with 5 μM glyceraldehyde for 15 minutes and titrated with quercetin. Ki in all the four cased were calculated strictly using equations mentioned in Example 11. The Data are shown in Table 8.

Table 8 : Summary of the dissociation constants of glyceraldehyde and quercetin with ALR2

| | Ki with ALR2 | Ki value in the presence of quercetin | Ki value in the presence of glyceraldehyde |
|---|---|---|---|
| Glyceraldehyde | 510 ± 54.0 μM | Not applicable | - |
| Quercetin | 164.0 ± 27.1 nM | - | 15.6 ± 2.1 nM |
| 1-Cyclohexylaldehyde | 330.1 ± 45.0 μM | 14.1 ± 3.1 nM | - |

**Example 15:**

**Dosage and treatment of bacterial infections:**

The drug administration and assays were conducted as per Sharma et al. (2003)Triclosan as a Systemic Antibacterial Agent in an Acute Bacterial Challenge Mouse Model, Antimicrob. Agents and Chemother. 12, 3859-3866 (2003) which is detailed below:-

Drug Administration: One group of animals was administered subcutaneous (in mid-dorsal region) or intraperitoneal Triclosan (40 mg/kg) in DMSO (25 μl) or Ethanol (25 μl). Another group of animals was administered 50 mg/kg of EGCG in 250-500 μl of PBS. Third group of animals received injections of both Triclosnan and ECGC as above. Controlled group was injected PBS and/or ethanol (500 μl).

For each experiment 6 BALB/c male mice, 6-9 week of age per group were used.
Infection of animals by bacteria: The animals were injected i.p or s.c with 0.4 ml of pyrogen free saline containing $10^7$ CFU of bacteria (*E.coli O55:B5*) and primed with D-galactosamine (300 mg/kg of body weight); (Galanos at al. (1979) Proc. Natl. Acad. Sci. USA, 76, 5939-5943).
The mice was monitored for survival on an hourly basis.

Beneficial effect of a flavinoid and triclosan treatment on survival of mice post bacterial infection

| | Treatment | Survival Time (hr) |
|---|---|---|
| 1. | Triclosan only | 45 ± 6 |
| 2. | Triclosan + ECGC | 54 ± 6 |
| 3. | PBS treated | 8 ± 2 |
| 4. | Ethanol treated | 8 ± 2 |
| 5. | PBS and Ethanol Treated | 8 ± 2 |

**REFERENCES**

1. Smith, S., Witkowski, A. and Joshi, A. K. (2003) Structural and functional organization of the animal fatty acid synthase. Prog, Lipid Res. 42, 289-317.

2. Surolia, N. and Surolia, A. (2001) Triclosan offers protection against blood stages of malaria by inhibiting enoyl-ACP reductase of P. falciparum: Nature Medicine 7, 167-173.

3. Heath, R. J, and Rock, C. O. (1995) Enoyl-acyl carrier protein reductase (fabl) plays a determinant role in completing cycles of fatty acid elongation in Escherichia coli. J. Biol. Chem. 270, 26538-26542.

4. Kapoor, M., Reddy, C. C., Krishnasastry, M. V., Surolia, N. and Surolia, A. (2004) Slow-tight-binding inhibition of enoyl-acyl carrier protein reductase from Plasmodium falciparum by triclosan. Biochem. J. 381, 719-24.

5. Kapoor, M., Mukhi, P. L., Surolia, N., Suguna, K. and Surolia, A. (2004) Kinetic and structural analysis of the increased affinity of enoyl-ACP (acyl-carrier protein) reductase for triclosan in the presence of NAD+. Biochem. J. 381, 725-733.

6. Pidugu, L. S., Kapoor, M., Surolia, N., Surolia, A., and Suguna, K. (2004) Structural basis for the variation in triclosan affinity to Enoyl Reductases. J. Mol. Biol. 343, 147-155.

7. Zhang, Y. M., and Rock, C. O. (2004) Evaluation of epigallocatechin gallate and related plant polyphenols as inhibitors of the FabG and Fabl reductases of bacterial type II fatty-acid synthase. J. Biol. Chem.279, 30994-1001.

8. Dixon, M. (1972), The Graphical Determination of Km and Ki. Biochem. J. 129, 197-202

9. Scatchard, G. (1949) The attraction of proteins for small molecules and ions. Ann. N. Y. Acad. Sci. 51, 660.

10. Morrison, J. F. and Walsh, C. T. (1988) The behavior and significance of slow-binding enzyme inhibitors. Adv. Enzymol. Relat. Areas Mol. Biol. 61, 201-301.

11. Morrison, J. F. (1982) The slow binding and slow-tight binding inhibition of enzyme catalyzed reactions. Trends Biochem. Sci. 7, 102-105.

12. McClerren, A. L., Endsley, S., Bowman, J. L., Anderson, N. H., Guan, Z., Rudolph, J. and Raetz, C. R. H. (2005) A slow, tight-binding inhibitor of the Zinc-dependent deacetylase LpxC of lipid, A biosynthesis with antibiotic activity comparable to Ciprofloxacin. Biochemistry, 44, 16474-16583

13. Houtzager, V., Oullet, M., Flagueyret, J. -P., Passmore,l. A., Bayly, C., and Percival, M. D. (1996) Inhibitor-induced changes in the intrinsic fluorescence of human cyclooxygenase-2. Biochemistry, 35, 10974-10984.

14. Lakowicz, J. R. (1983) Principles of Fluorescence Spectroscopy, pp, 180-195, Plenum Press, New York

15. Morris, G. M., Goodsell, D. S., Halliday, R.S., Huey, R., Hart, W. E., Belew, R. K. and Olson, A. J. (1998) "Automated Docking Using a Lamarckian Genetic Algorithm and Empirical Binding Free Energy Function" J. Comp. Chem. 19, 1639-1662.

16. Molecular Operating Environment [MOE 2001.07], Chemical Computing Group Inc.,1255 University St., Suite 1600, Montreal, Quebec, Canada, H3B 3X3.

17. Perozzo, R., Kuo, M., Sidhu, A. S., Valiyaveettil, J. T., Bittman, R., Jacobs, W. R. Jr., Fidock, D. A., Sacchettini, J. C. (2002) Structural Elucidation of the Specificity of the Antibacterial Agent Triclosan for Malarial Enoyl ACP Reductase. J. Biol. Chem. 277, 13106-13114.

18. Stewart, M. J., Parikh, S., Xiao, G., Tonge, P. J., and Kisker, C. (1999) Structural Basis and Mechanism of Enoyl Reductase Inhibition by Triclosan. J. Mol. Biol. 290, 859-65.

19. Mehler, E. L., and Solmajer, T. (1991) Electrostatic effects in proteins: comparison of dielectric and charge models. Protein Eng. 4, 903-910.

20. Chung, S., and LaMendola, J. (1989) Cloning and sequence determination of human placental aldose reductase gene. J. Biol. Chem. 264(25), 14775-14777.

21. Tarle, I., Borhani, D. W., Wilson, D.K., Quiocho, F.A., and Petrash, J.M. (1993) Probing the active site of human aldose reductase. Site-directed mutagenesis of Asp-43, Tyr-48, Lys-77, and His-110. J. Biol. Chem. 268(34), 25687-25693.

22. Kapoor, M., Dar, M. J., Surolia, A. and Surolia, N. (2001) Kinetic determination of the interaction of Enoyl-ACP Reductase from Plasmodium falciparum with its substrates and inhibtitors. Biochem. Biophys. Res. Commun. 289, 832-837.

23. Sharma, S.K., Parasuraman, P., Kumar, G., Surolia, N. and Surolia, A., (2007) Green Tea Catechins Potentiate Triclosan Binding to Enoyl-ACP Reductase from Plasmodium falciparum (PfENR). J. Med. Chem. 50, 765-775.

24. Tasdemir, D., Lack, G., Brun, R., Ruedi, P., Scapozza, L. and Perozzo, R. (2006) Inhibition of Plasmodium falciparum Fatty Acid Biosynthesis: Evaluation of FabG, FabZ, and FabI as Drug Targets for Flavonoids, J. Med. Chem. 49, 3345-3353.

**Claims**

1. A composition useful for enhancing effect of an inhibitor in inhibiting $NAD^+$/$NADP^+$ or NADH/NADPH dependent enzymes, said composition comprising an effective amount of inhibitor and a polyphenol.

2. The composition as claimed in claim 1, wherein the inhibitor is 2'-hydroxydiaryl ether or derivatives thereof.

3. The composition as claimed in claim 1, wherein the inhibitor is 2'-hydroxydiphenyl ether or derivatives thereof.

4. The composition as claimed in claim 1, wherein the inhibitor is triclosan.

5. The composition as claimed in claim 1, wherein the polyphenol is selected from a group consisting of flavonoid and buteine.

6. The composition as claimed in claim 5, wherein the flavonoid is selected from a group consisting of flavonol, isoflavonol, flavanones, flavan-3-ol and derivatives thereof.

7. The composition as claimed in claim 5, wherein the flavonoid is quercetin.

8. The composition as claimed in claim 6, wherein the flavan-3-ol is catechin.

9. The composition as claimed in claim 8, wherein the catechin is selected from a group consisting of epigallocatechin gallete (EGCG), epigallocatechin (EGC) and epicatechin gallete (ECG).

10. The composition as claimed in claim 1 further comprising at least one of a pharmaceutically acceptable adjuvant, carrier, diluent or excipient.

11. The composition as claimed in claim 1 further comprising an antimicrobial compound.

12. The composition as claimed in claim 11, wherein the antimicrobial compound is selected from a group consisting of antibacterial compound, antimalarial compound, antiprotozoal compound, antifungal compound, antiproliferative compound and analgesic.

13. The composition according to any one of claims 1 to 12 in a form suitable for parenteral administration.

14. The composition as claimed in claim 1, wherein the $NAD^+$/$NADP^+$ or NADH/NADPH dependent enzymes are selected

from a group consisting of *Plasmodium falciparum* enoyl-Acyl carrier protein (ACP) reductase (PfENR), *E. coli* enoyl-Acyl carrier protein (ACP) reductase (EcENR), aldolase reductase, steroid 5α-reductase, P. *falciparum* FabG , bacterial FabG, dihydrofolate reductase and squalene epoxidase.

**15.** The composition as claimed in claim 1, wherein the $NAD^+/NADP^+$ or NADH/NADPH dependent enzyme is PfENR.

**16.** The composition as claimed in claim 1, wherein the $NAD^+/NADP^+$ or NADH/NADPH dependent enzyme is EcENR.

**17.** The composition as claimed in claim 1, wherein the $NAD^+/NADP^+$ or NADH/NADPH dependent enzymes belong to infectious agents selected from a group consisting of *Plasmodium species, bacterial species* and an *Apicomplexa species.*

**18.** The composition as claimed in claim 17, wherein the *Plasmodium species* is selected from a group consisting of *Plasmodium falciparum, Plasmodium vivax, Plasmodium ovale* and *Plasmodium malariae.*

**19.** The composition as claimed in claim 17, wherein the *Plasmodium species* is *Plasmodium falciparum.*

**20.** The composition as claimed in claim 17, wherein the *bacterial* species is selected from a group consisting of *Escherichia coli and Mycobacterium tuberculosis.*

**21.** The composition as claimed in claim 17, wherein the *bacterial* species is *Escherichia coli.*

**22.** A composition useful for enhancing effect of an inhibitor in inhibiting aldose reductase, said composition comprising effective amount of inhibitor and a polyphenol.

**23.** The composition as claimed in claim 22, wherein the inhibitor is sugar aldehyde, preferably glycerladehyde.

**24.** The composition as claimed in claim 22, wherein the inhibitor an aldehyde such as 1-cyclohexylaldehyde

**25.** The composition as claimed in claim 22, wherein the polyphenol is selected from a group consisting of flavonoid and buteine.

**26.** The composition as claimed in claim 25 wherein the flavonoid is selected from a group consisting of flavonol, isoflavonol, flavanones, flavan-3-ol and derivatives thereof.

**27.** The composition as claimed in claim 25, wherein the flavonoid is quercetin.

**28.** The composition as claimed in claim 26, wherein the flavan-3-ol is catechin.

**29.** The composition as claimed in claim 28, wherein the catechin is selected from a group consisting of epigallocatechin gallete (EGCG), epigallocatechin (EGC) and epicatechin gallete (ECG).

**30.** The composition as claimed in claim 22, wherein the composition is useful for the treatment of complications of diabetes.

**31.** The composition as claimed in claim 30, wherein the complications of diabetes are selected from a group consisting of diabetic retinopathy, cataract neuropathy and neural complication.

**32.** Use of a composition comprising triclosan and EGCG for preparing a medicament for treatment of malaria.

**33.** Use of a composition comprising triclosan and ECGC for preparing a medicament for treating a bacterial infection.

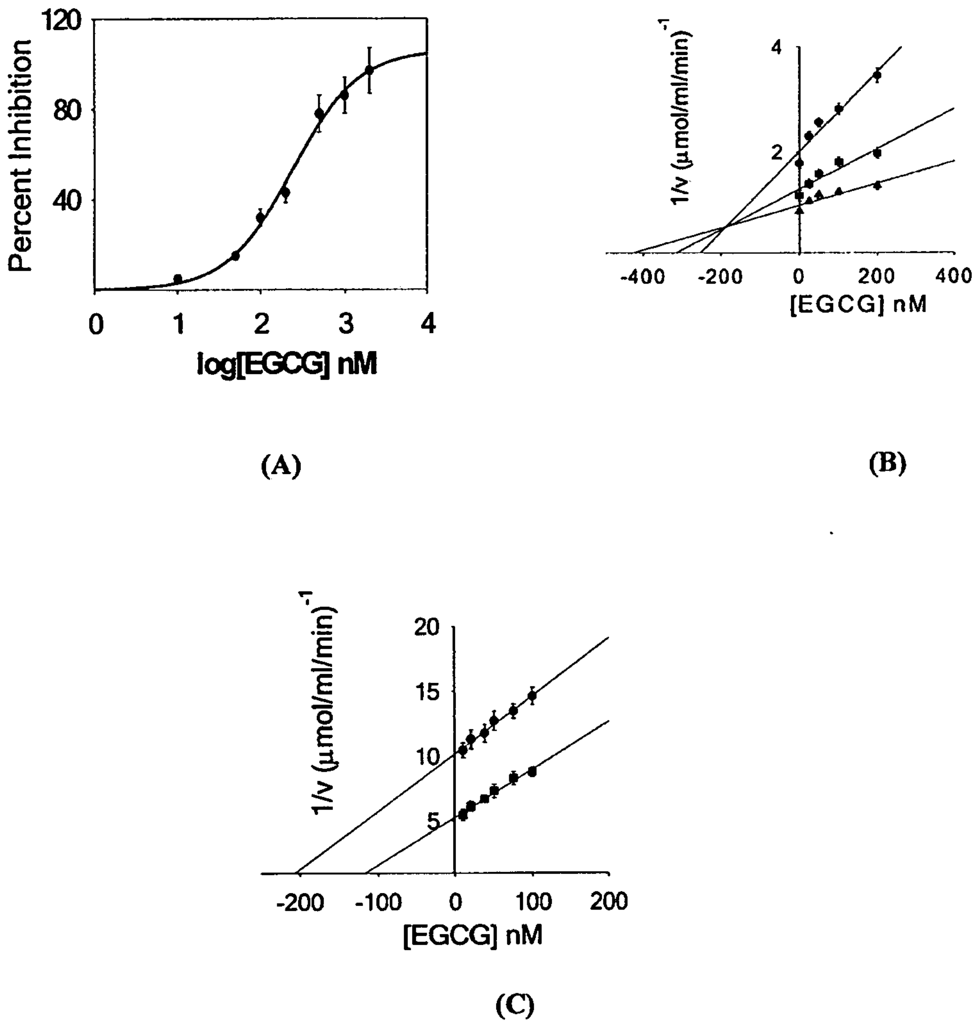
120
80
40
Percent Inhibition
0
1
2
3
4
log[EGCG] nM
(A)
1/v (μmol/ml/min)-1
4
2
-400
-200
0
200
400
[EGCG] nM
(B)
1/v (μmol/ml/min)-1
20
15
10
5
-200
-100
0
100
200
[EGCG] nM
(C)

**Figure 1**

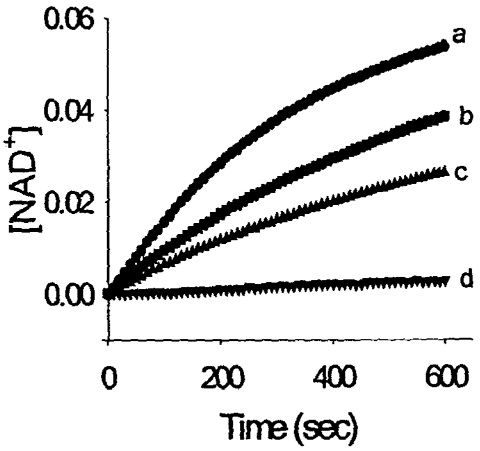


Figure 2

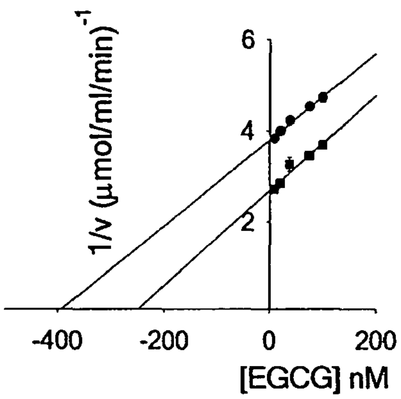
1/v (μmol/ml/min)-1
6
4
2
-400
-200
0
200
[EGCG] nM

(A)

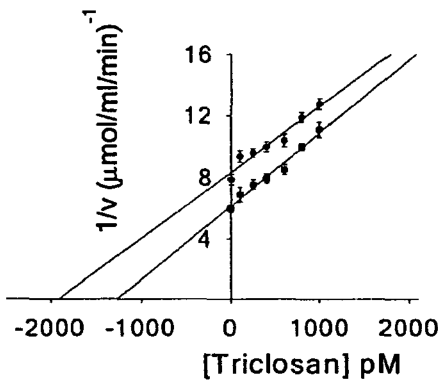
1/v (μmol/ml/min)-1
16
12
8
4
-2000
-1000
0
1000
2000
[Triclosan] pM

(B)

Figure 3

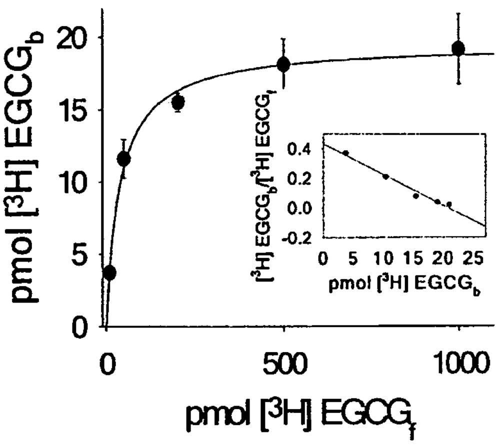
pmol [3H] EGCG_b
pmol [3H] EGCG_f
[3H] EGCG_b/[3H] EGCG_f
pmol [3H] EGCG_b

(A)

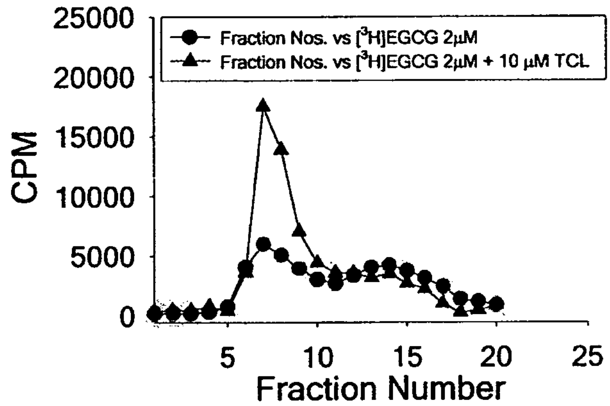
Fraction Nos. vs [3H]EGCG 2μM
Fraction Nos. vs [3H]EGCG 2μM + 10 μM TCL
CPM
Fraction Number

(B)

Figure 4

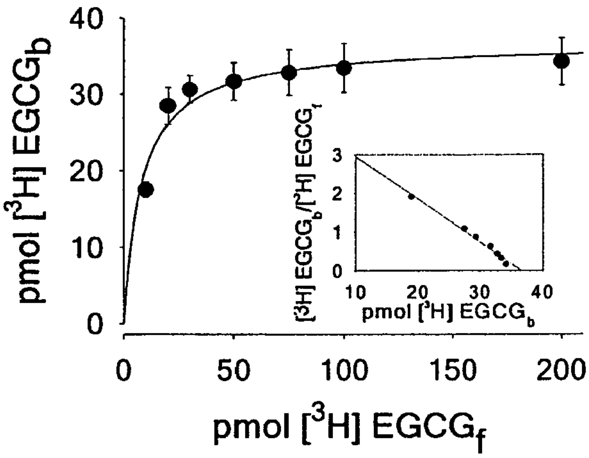


(C)

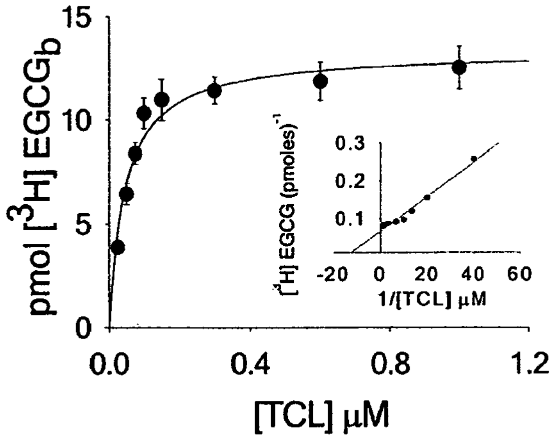


Figure 4

(D)

Figure 4

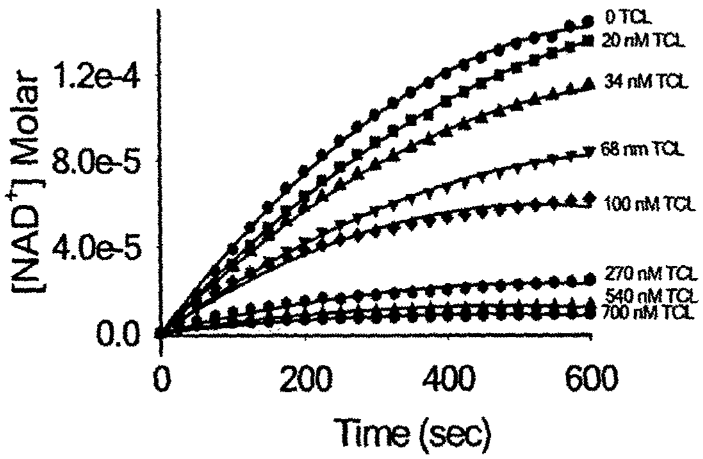


**Figure 5**

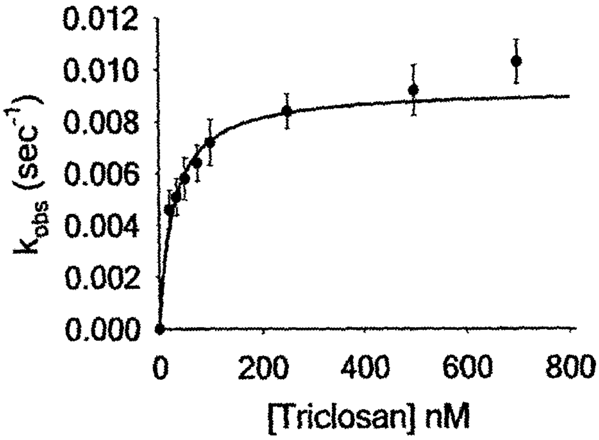


**Figure 6**

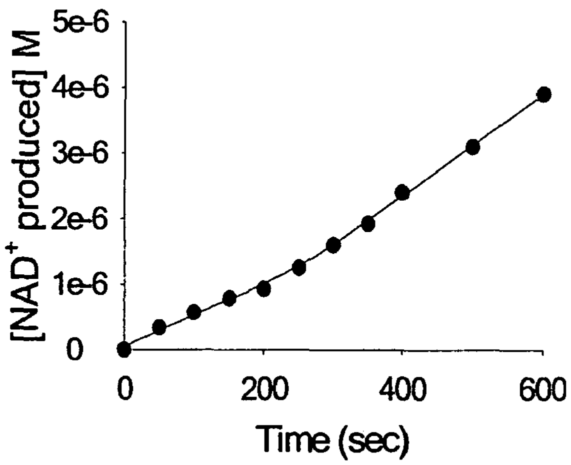


**Figure 7**

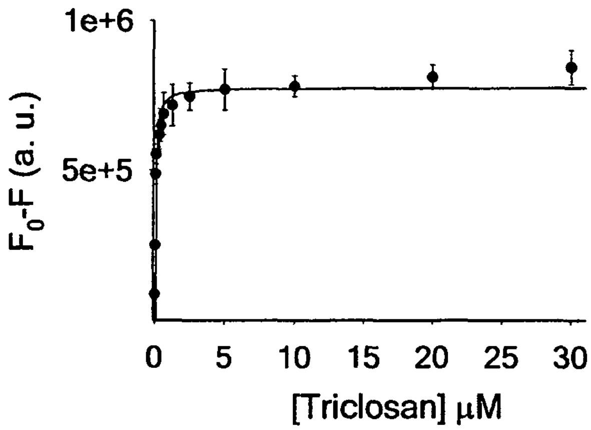


**Figure 8**

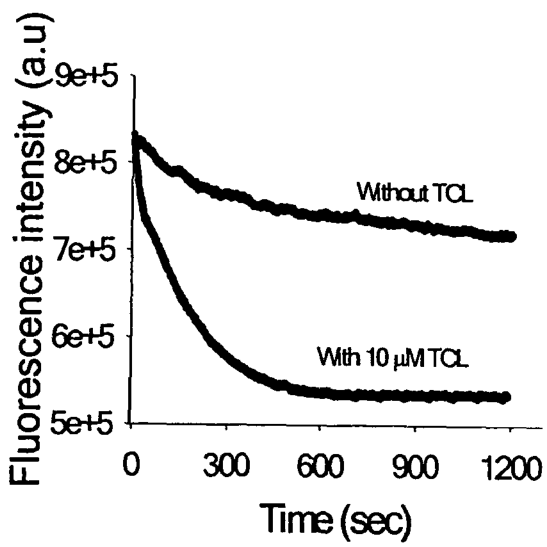
Fluorescence intensity (a.u)
9e+5
8e+5
7e+5
6e+5
5e+5
0
300
600
900
1200
Time (sec)
Without TCL
With 10 μM TCL

**Figure 9**

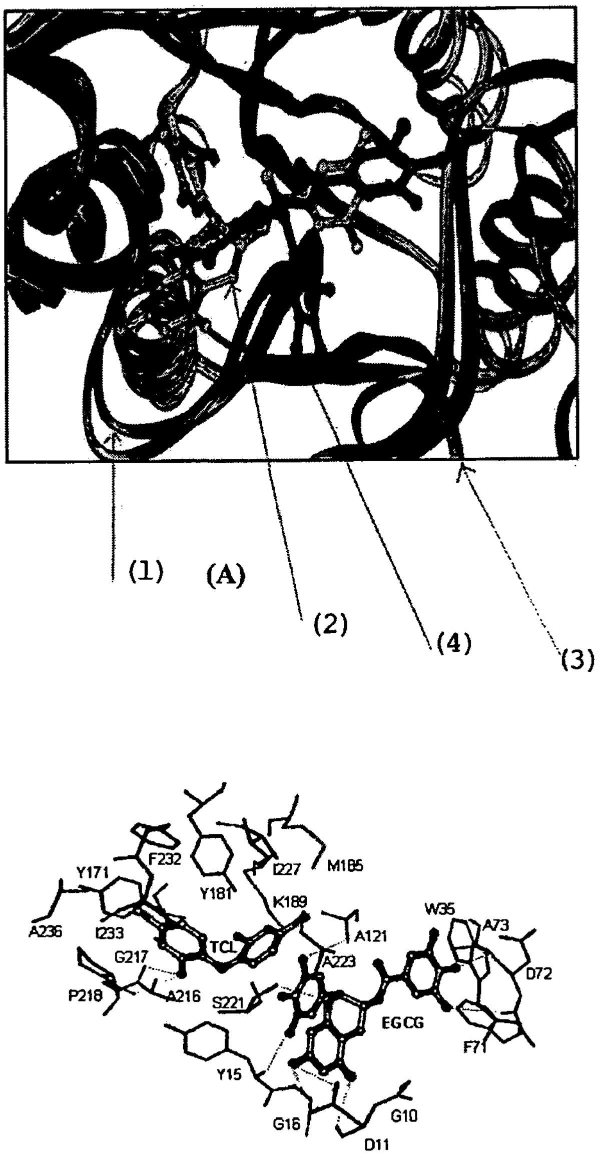
(1)
(2)
(4)
(3)
Y171
F232
I227
M185
Y181
K189
A236
I233
TCL
A121
W35
A73
G217
A223
D72
P218
A216
S221
EGCG
F71
Y15
G16
G10
D11

(A)

(B)

**Figure 10**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SUROLIA ; SUROLIA.** *Nature Medicine,* 2001, vol. 7, 167-173
- **SHARMA et al.** Triclosan as a Systemic Antibacterial Agent in an Acute Bacterial Challenge Mouse Model. *Antimicrob. Agents and Chemother.,* 2003, vol. 12, 3859-3866
- **GALANOS.** *Proc. Natl. Acad. Sci. USA,* 1979, vol. 76, 5939-5943
- **SMITH, S. ; WITKOWSKI, A ; JOSHI, A. K.** Structural and functional organization of the animal fatty acid synthase. *Prog, Lipid Res.,* 2003, vol. 42, 289-317
- **SUROLIA, N ; SUROLIA, A.** Triclosan offers protection against blood stages of malaria by inhibiting enoyl-ACP reductase of P. falciparum. *Nature Medicine,* 2001, vol. 7, 167-173
- **HEATH, R. J ; ROCK, C. O.** Enoyl-acyl carrier protein reductase (fabl) plays a determinant role in completing cycles of fatty acid elongation in Escherichia coli. *J. Biol. Chem.,* 1995, vol. 270, 26538-26542
- **KAPOOR, M ; REDDY, C. C ; KRISHNASASTRY, M. V ; SUROLIA, N ; SUROLIA, A.** Slow-tight-binding inhibition of enoyl-acyl carrier protein reductase from Plasmodium falciparum by triclosan. *Biochem. J.,* 2004, vol. 381, 719-24
- **KAPOOR, M ; MUKHI, P. L ; SUROLIA, N ; SUGUNA, K ; SUROLIA, A.** Kinetic and structural analysis of the increased affinity of enoyl-ACP (acyl-carrier protein) reductase for triclosan in the presence of NAD+. *Biochem. J.,* 2004, vol. 381, 725-733
- **PIDUGU, L. S ; KAPOOR, M ; SUROLIA, N ; SUROLIA, A ; SUGUNA, K.** Structural basis for the variation in triclosan affinity to Enoyl Reductases. *J. Mol. Biol.,* 2004, vol. 343, 147-155
- **ZHANG, Y. M ; ROCK, C. O.** Evaluation of epigallocatechin gallate and related plant polyphenols as inhibitors of the FabG and Fabl reductases of bacterial type II fatty-acid synthase. *J. Biol. Chem.,* 2004, vol. 279, 30994-1001
- **DIXON, M.** The Graphical Determination of Km and Ki. *Biochem. J.,* 1972, vol. 129, 197-202
- **SCATCHARD, G.** The attraction of proteins for small molecules and ions. *Ann. N. Y. Acad. Sci.,* 1949, vol. 51, 660
- **MORRISON, J. F ; WALSH, C. T.** The behavior and significance of slow-binding enzyme inhibitors. *Adv. Enzymol. Relat. Areas Mol. Biol.,* 1988, vol. 61, 201-301
- **MORRISON, J. F.** The slow binding and slow-tight binding inhibition of enzyme catalyzed reactions. *Trends Biochem. Sci.,* 1982, vol. 7, 102-105
- **MCCLERREN, A. L ; ENDSLEY, S ; BOWMAN, J. L ; ANDERSON, N. H ; GUAN, Z ; RUDOLPH, J ; RAETZ, C. R. H.** A slow, tight-binding inhibitor of the Zinc-dependent deacetylase LpxC of lipid, A biosynthesis with antibiotic activity comparable to Ciprofloxacin. *Biochemistry,* 2005, vol. 44, 16474-16583
- **HOUTZAGER, V ; OULLET, M ; FLAGUEYRET, J. -P ; PASSMORE,L. A ; BAYLY, C ; PERCIVAL, M. D.** Inhibitor-induced changes in the intrinsic fluorescence of human cyclooxygenase-2. *Biochemistry,* 1996, vol. 35, 10974-10984
- **LAKOWICZ, J. R.** Principles of Fluorescence Spectroscopy. Plenum Press, 1983, 180-195
- **MORRIS, G. M ; GOODSELL, D. S ; HALLIDAY, R.S ; HUEY, R ; HART, W. E ; BELEW, R. K ; OLSON, A. J.** Automated Docking Using a Lamarckian Genetic Algorithm and Empirical Binding Free Energy Function. *J. Comp. Chem.,* 1998, vol. 19, 1639-1662
- Molecular Operating Environment [MOE 2001.07. Chemical Computing Group Inc
- **PEROZZO, R ; KUO, M ; SIDHU, A. S ; VALIYAVEETTIL, J. T ; BITTMAN, R ; JACOBS, W. R. JR ; FIDOCK, D. A ; SACCHETTINI, J. C.** Structural Elucidation of the Specificity of the Antibacterial Agent Triclosan for Malarial Enoyl ACP Reductase. *J. Biol. Chem.,* 2002, vol. 277, 13106-13114
- **STEWART, M. J ; PARIKH, S ; XIAO, G ; TONGE, P. J ; KISKER, C.** Structural Basis and Mechanism of Enoyl Reductase Inhibition by Triclosan. *J. Mol. Biol.,* 1999, vol. 290, 859-65
- **MEHLER, E. L ; SOLMAJER, T.** Electrostatic effects in proteins: comparison of dielectric and charge models. *Protein Eng.,* 1991, vol. 4, 903-910
- **CHUNG, S ; LAMENDOLA, J.** Cloning and sequence determination of human placental aldose reductase gene. *J. Biol. Chem.,* 1989, vol. 264 (25), 14775-14777
- **TARLE, I ; BORHANI, D. W ; WILSON, D.K ; QUIOCHO, F.A ; PETRASH, J.M.** Probing the active site of human aldose reductase. Site-directed mutagenesis of Asp-43, Tyr-48, Lys-77, and His-110. *J. Biol. Chem.,* 1993, vol. 268 (34), 25687-25693

- **KAPOOR, M ; DAR, M. J ; SUROLIA, A ; SUROLIA, N.** Kinetic determination of the interaction of Enoyl-ACP Reductase from Plasmodium falciparum with its substrates and inhibtitors. *Biochem. Biophys. Res. Commun.,* 2001, vol. 289, 832-837
- **SHARMA, S.K ; PARASURAMAN, P ; KUMAR, G ; SUROLIA, N ; SUROLIA, A.** Green Tea Catechins Potentiate Triclosan Binding to Enoyl-ACP Reductase from Plasmodium falciparum (PfENR. *J. Med. Chem.,* 2007, vol. 50, 765-775
- **TASDEMIR, D ; LACK, G ; BRUN, R ; RUEDI, P ; SCAPOZZA, L ; PEROZZO, R.** Inhibition of Plasmodium falciparum Fatty Acid Biosynthesis: Evaluation of FabG, FabZ, and Fabl as Drug Targets for Flavonoids. *J. Med. Chem.,* 2006, vol. 49, 3345-3353